(19) Europäisches Patentamt
European Patent Office
Office européen des brevets

(11) **EP 4 389 865 A1**

(12) **EUROPEAN PATENT APPLICATION**

(43) Date of publication:
**26.06.2024 Bulletin 2024/26**

(21) Application number: **22215666.3**

(22) Date of filing: **21.12.2022**

(51) International Patent Classification (IPC):
***C11D 3/386*** (2006.01)   ***C12N 5/07*** (2010.01)
***C12N 9/50*** (2006.01)

(52) Cooperative Patent Classification (CPC):
**C12N 5/0696; C12N 5/0657; C12N 9/6408;
C12N 9/6416; C12Y 304/21001; C12Y 304/21004;
C12Y 304/24003; C12Y 304/24024;
C12Y 304/24035**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Novozymes A/S
2880 Bagsvaerd (DK)**
• **Novo Nordisk Pharmatech A/S
4600 Køge (DK)**

(72) Inventors:
• **HOFF, Tine
2880 Bagsvaerd (DK)**
• **JOERGENSEN, Christian, Isak
2880 Bagsvaerd (DK)**
• **SALOMON, Jesper
2880 Bagsvaerd (DK)**
• **KRISTENSEN, Karina, Sandgaard
4600 Koege (DK)**
• **REISER, Anna, Verena
4600 Koege (DK)**

(74) Representative: **NZ EPO Representatives
Krogshoejvej 36
2880 Bagsvaerd (DK)**

Remarks:
The complete document including Reference
Table(s) and the Sequence Listing(s) can be
downloaded from the EPO website

(54) **RECOMBINANT PROTEASE FOR CELL DETACHMENT**

(57) The present invention relates to compositions suitable for cell detachment comprising polypeptides with protease activity. The present invention further relates to polypeptides with protease activity, polynucleotides encoding said polypeptides, nucleic acid constructs and expression vectors comprising said polynucleotides, recombinant host cells comprising said nucleic acid constructs or expressions vectors, methods of producing said polypeptides, granules comprising said polypeptides, fermentation broth formulations comprising said polypeptides, and methods and uses employing said polypeptides.

EP 4 389 865 A1

**Description**

**Reference to sequence listing**

This application contains a Sequence Listing in computer readable form. The computer readable form is incorporated herein by reference.

**FIELD OF THE INVENTION**

The present invention relates to compositions suitable for cell detachment comprising polypeptides with protease activity. The present invention further relates to polypeptides with protease activity, polynucleotides encoding said polypeptides, nucleic acid constructs and expression vectors comprising said polynucleotides, recombinant host cells comprising said nucleic acid constructs or expressions vectors, methods of producing said polypeptides, granules comprising said polypeptides, fermentation broth formulations comprising said polypeptides, and methods and uses employing said polypeptides.

**BACKGROUND OF THE INVENTION**

Cell detachment is a critical step during passaging of cells when grown as adherent cells as well as cell clusters. The detachment step preferably involves the use of proteolytic enzymes as these are mild yet effective in terms of releasing the cells from a surface to which they adhere as well as dissolving cell clusters formed in suspension cultures.

Accutase® and Accumax® (both available from, e.g., Innovative Cell Technologies, Inc.) are commercially available products for cell detachment that include a mixture of enzymes with proteolytic and collagenolytic activity that are isolated from an invertebrate source. A disadvantage associated with these products is that regulatory authorities generally do not allow animal-derived products to be used in drug development and production processes, which hampers their applicability in development and production of cell-based therapies. Another disadvantage of these products is the inherent risk batch-to-batch variation in terms of composition and activity as a consequence of the products being isolated from an animal source.

TrypLE™ (available from, e.g., ThermoFisher Scientific) is a commercially available trypsin product that may be used for cell detachment. TrypLE™ is produced recombinantly and is thus not of animal origin. However, a disadvantage associated with TrypLE™ is that not all types of cells are sufficiently detached when subjected to trypsin treatment alone, which limits the broad applicability of this product.

An object of the present invention is to provide an enzymatic solution that addresses the drawbacks associated with current products for cell detachment. In particular, an object of the present invention is to provide an enzymatic solution that is compatible with regulatory requirements and useful in detachment of many different cell types and may be produced in a uniform manner with no batch-to-batch variation.

**SUMMARY OF THE INVENTION**

The present invention provides a recombinantly expressed alternative to the animal-derived enzymatic products that are currently being used for cell detachment processes. The present inventors have analysed the Accutase® product and identified a single protease (SEQ ID NO: 1) as being the main contributor to cell detachment. SEQ ID NO: 1 has an increased P1 preference for the amino acid residues Leu, Tyr, and Phe. Without being bound by theory, it is speculated that the P1 preference profile exhibited by SEQ ID NO: 1 provides an effective yet mild cleavage of cell surface proteins involved in surface attachment and cell-cell adhesion.

In a first aspect, the present invention relates to a composition suitable for cell detachment comprising a polypeptide having protease activity and a sequence identity of at least 70% to SEQ ID NO: 1, wherein said polypeptide is present in an amount of at least 50% by weight of the total polypeptide material of the composition.

In a second aspect, the present invention relates to an isolated polynucleotide encoding a polypeptide having at least 70% sequence identity to SEQ ID NO: 1.

In a third aspect, the present invention relates to a nucleic acid construct or an expression vector comprising a polynucleotide encoding a polypeptide having at least 70% sequence identity to SEQ ID NO: 1 operably linked to a heterologous promoter.

In a fourth aspect, the present invention relates to a recombinant host cell comprising in its genome the nucleic acid construct or expression vector of the third aspect.

In a fifth aspect, the present invention relates to a method of producing a polypeptide having a sequence identity of at least 70% to SEQ ID NO: 1 and having protease activity, comprising (a) cultivating a recombinant host cell according to the fourth aspect under conditions conducive for expression of the polypeptide; and optionally, (b) recovering the polypeptide.

In a sixth aspect, the present invention relates to a fermentation broth formulation comprising a polypeptide having a sequence identity of at least 70% to SEQ ID NO: 1 and having protease activity.

In a seventh aspect, the present invention relates to a method for cell detachment, comprising contacting a cell with a composition according to the first aspect, wherein the cell is attached to a surface and/or to another cell.

In an eighth aspect, the present invention relates to use of a composition of the first aspect in a cell detachment process.

## BRIEF DESCRIPTION OF DRAWINGS

Fig. 1 shows SDS-PAGE analysis of collagen type IV degradation. Lane 1: protein ladder. Lane 2: desalted Accutase at 0.44 μg/mL with added collagen type IV substrate. Lane 3: desalted Accutase at 0.1 μg/mL with added collagen type IV substrate. Lane 4: desalted Accutase without substrate. Lane 5: collagen type IV substrate only.

Fig. 2 shows a schematic overview of the hPSC setup with an indication of when evaluation of hPSC monolayer detachment and cluster formation is performed (circled passage).

Fig. 3 shows a schematic overview of the hPSC setup with an indication of when evaluation of hPSC cluster dissociation and re-formation is performed (circled passage).

## SEQUENCE OVERVIEW

SEQ ID NO: 1 is an S1 protease isolated from the Accutase® product.

SEQ ID NO: 2 is a codon-optimized DNA sequence gene encoding the S1 protease of SEQ ID NO: 1.

SEQ ID NO: 3 is the beta-glucosidase secretion signal from *Aspergillus aculeatus*.

## DEFINITIONS

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon, such as ATG, GTG, or TTG, and ends with a stop codon, such as TAA, TAG, or TGA. The coding sequence may be a genomic DNA, cDNA, synthetic DNA, or a combination thereof.

**Control sequences:** The term "control sequences" means nucleic acid sequences involved in regulation of expression of a polynucleotide in a specific organism or in vitro. Each control sequence may be native (*i.e.*, from the same gene) or heterologous (i.e., from a different gene) to the polynucleotide encoding the polypeptide, and native or heterologous to each other. Such control sequences include, but are not limited to leader, polyadenylation, prepropeptide, propeptide, signal peptide, promoter, terminator, enhancer, and transcription or translation initiator and terminator sequences. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" means any step involved in the production of a polypeptide including, but not limited to, transcription, post-transcriptional modification, translation, post-translational modification, and secretion.

**Expression vector:** An "expression vector" refers to a linear or circular DNA construct comprising a DNA sequence encoding a polypeptide, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

**Heterologous:** The term "heterologous" means, with respect to a host cell, that a polypeptide or nucleic acid does not naturally occur in the host cell. The term "heterologous" means, with respect to a polypeptide or nucleic acid, that a control sequence, e.g., promoter, of a polypeptide or nucleic acid is not naturally associated with the polypeptide or nucleic acid, *i.e.,* the control sequence is from a gene other than the gene encoding the mature polypeptide.

**Host Strain or Host Cell:** A "host strain" or "host cell" is an organism into which an expression vector, phage, virus, or other DNA construct, including a polynucleotide encoding a polypeptide of the present invention has been introduced. Exemplary host strains are microorganism cells (*e.g.*, bacteria, filamentous fungi, and yeast) capable of expressing the polypeptide of interest and/or fermenting saccharides. The term "host cell" includes protoplasts created from cells.

**Isolated:** The term "isolated" means a polypeptide, nucleic acid, cell, or other specified material or component that has been separated from at least one other material or component, including but not limited to, other proteins, nucleic acids, cells, etc. An isolated polypeptide, nucleic acid, cell or other material is thus in a form that does not occur in nature. An isolated polypeptide includes, but is not limited to, a culture broth containing the secreted polypeptide expressed in a host cell.

**Native:** The term "native" means a nucleic acid or polypeptide naturally occurring in a host cell.

**Nucleic acid:** The term "nucleic acid" encompasses DNA, RNA, heteroduplexes, and synthetic molecules capable of encoding a polypeptide. Nucleic acids may be single stranded or double stranded and may be chemically modified. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature, or which is synthetic, and which comprises one or more control sequences operably linked to the nucleic acid sequence.

**Operably linked:** The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequence.

**Passage:** The term "passage" refers to the process of removing some or all cells from a culture and transferring the cells into fresh growth medium. Passaging of cells may also be referred to as subculturing. In some embodiments, passaging leads to a single cell suspension.

**Protease:** The term "protease" means an enzyme having peptidase activity (EC 3.4; also known as protease activity) that catalyzes the hydrolysis of peptide bonds. The EC 3.4 group includes several sub-groups, including EC 3.4.21 (serine endopeptidase), which further contains several sub-groups, including EC. 3.4.21.62 (subtilisin). The term "protease" and the expression "polypeptide having protease activity" are used interchangeably herein.

For purpose of the present invention, protease activity (E.C. 3.4) may be determined according to the Protease Activity Assay described in the Examples herein.

For purpose of the present invention, trypsin activity (EC 3.4.21.4) may be determined according to the Trypsin Activity Assay described in the Examples herein.

For purpose of the present invention, chymotrypsin activity (EC 3.4.21.1) may be determined according to the Chymotrypsin Activity Assay described in the Examples herein.

For purpose of the present invention, collagenase type I activity may be determined according to the Collagenase Type I Activity Assay described in the Examples herein.

For purpose of the present invention, collagenase type IV activity may be determined according to the Collagenase Type IV Activity Assay described in the Examples herein.

**Purified:** The term "purified" means a nucleic acid, polypeptide (e.g., protease) or cell that is substantially free from other components as determined by analytical techniques well known in the art (e.g., a purified polypeptide or nucleic acid may form a discrete band in an electrophoretic gel, chromatographic eluate, and/or a media subjected to density gradient centrifugation). A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8%, about 99.9%, or more, pure (e.g., percent by weight or on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

In one aspect, the term "purified" as used herein refers to the polypeptide (e.g., protease) or cell being essentially free from components (especially insoluble components) from the production organism. In other aspects. the term "purified" refers to the polypeptide being essentially free of insoluble components (especially insoluble components) from the native organism from which it is obtained. In one aspect, the polypeptide is separated from some of the soluble components of the organism and culture medium from which it is recovered. The polypeptide may be purified (*i.e.*, separated) by one or more of the unit operations filtration, precipitation, or chromatography.

Accordingly, the polypeptide (*e.g.*, protease) may be purified such that only minor amounts of other proteins, in particular, other polypeptides, are present. The term "purified" as used herein may refer to removal of other components, particularly other proteins and most particularly other enzymes present in the cell of origin of the polypeptide. The polypeptide may be "substantially pure", *i.e.*, free from other components from the organism in which it is produced, *e.g.*, a host organism for recombinantly produced polypeptide. In one aspect, the polypeptide is at least 40% pure by weight of the total polypeptide material present in the preparation. In one aspect, the polypeptide is at least 50%, 60%, 70%, 80% or 90% pure by weight of the total polypeptide material present in the preparation (*e.g.*, composition suitable for cell detachment). As used herein, a "substantially pure polypeptide" may denote a polypeptide preparation that contains at most 10%, preferably at most 9%, preferably at most 8%, preferably at most 7%, more preferably at most 6%, more preferably at most 5%, more preferably at most 4%, more preferably at most 3%, more preferably at most 2%, more preferably at most 1%, more preferably at most 0.5, more preferably at most 0.1%, more preferably at most 0.05%, more preferably at most 0.01%, even more preferably at most 0.005%, and most preferably at most 0.001% by weight of other polypeptide material with which the polypeptide is natively or recombinantly associated.

It is, therefore, preferred that the substantially pure polypeptide (*e.g.*, protease) is at least 90% pure, preferably at least 91%, more preferably at least 92% pure, more preferably at least 93% pure, more preferably at least 94% pure, more preferably at least 95% pure, more preferably at least 96% pure, more preferably at least 97% pure, more preferably at least 98% pure, more preferably at least 99% pure, more preferably at least 99.5% pure, more preferably at least 99.9% pure, more preferably at least 99.95%, more preferably at least 99.99% pure, even more preferably at least 99.995% pure, and most preferably at least 99.999% pure by weight of the total polypeptide material present in the preparation (*e.g.*, composition suitable for cell detachment). The polypeptide of the present invention is preferably in a substantially pure form (i.e., the preparation is essentially free of other polypeptide material with which it is natively or recombinantly associated). This can be accomplished, for example by preparing the polypeptide by well-known recombinant methods or by classical purification methods.

**Recombinant:** The term "recombinant" is used in its conventional meaning to refer to the manipulation, *e.g.*, cutting and rejoining, of nucleic acid sequences to form constellations different from those found in nature. The term recombinant refers to a cell, nucleic acid, polypeptide or vector that has been modified from its native state. Thus, for example, recombinant cells express genes that are not found within the native (non-recombinant) form of the cell, or express native genes at different levels or under different conditions than found in nature. The term "recombinant" is synonymous with "genetically modified" and "transgenic".

**Recover:** The terms "recover" or "recovery" means the removal of a polypeptide from at least one fermentation broth component selected from the list of a cell, a nucleic acid, or other specified material, *e.g.*, recovery of the polypeptide from the whole fermentation broth, or from the cell-free fermentation broth, by polypeptide crystal harvest, by chromatography, by filtration, *e.g.*, depth filtration (by use of filter aids or packed filter medias, cloth filtration in chamber filters, rotary-drum filtration, drum filtration, rotary vacuum-drum filters, candle filters, horizontal leaf filters or similar, using sheet or pad filtration in framed or modular setups) or membrane filtration (using sheet filtration, module filtration, candle filtration, microfiltration, ultrafiltration in either cross flow, dynamic cross flow or dead end operation), or by centrifugation (using decanter centrifuges, disc stack centrifuges, hydro cyclones or similar), or by precipitating the polypeptide and using relevant solid-liquid separation methods to harvest the polypeptide from the broth media by use of classification separation by particle sizes. Recovery encompasses isolation and/or purification of the polypeptide.

**Sequence identity:** The relatedness between two amino acid sequences or between two nucleotide sequences is described by the parameter "sequence identity".

For purposes of the present invention, the sequence identity between two amino acid sequences is determined as the output of "longest identity" using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. In order for the Needle program to report the longest identity, the -nobrief option must be specified in the command line. The output of Needle labeled "longest identity" is calculated as follows:

$$\text{(Identical Residues} \times 100)/(\text{Length of Alignment} - \text{Total Number of Gaps in Alignment)}$$

For purposes of the present invention, the sequence identity between two polynucleotide sequences is determined as the output of "longest identity" using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 6.6.0 or later. The parameters used are a gap open penalty of 10, a gap extension penalty of 0.5, and the EDNAFULL (EMBOSS version of NCBI NUC4.4) substitution matrix. In order for the Needle program to report the longest identity, the nobrief option must be specified in the command line. The output of Needle labeled "longest identity" is calculated as follows:

(Identical Deoxyribonucleotides x 100)/(Length of Alignment – Total Number of Gaps in Alignment)

## DETAILED DESCRIPTION OF THE INVENTION

The present invention provides a recombinantly expressed alternative to the animal-derived enzymatic products that are currently being used for cell detachment processes. The present inventors have analysed the Accutase® product and identified a single protease (SEQ ID NO: 1) as being the main contributor to cell detachment. SEQ ID NO: 1 has an increased P1 preference for the amino acid residues Leu, Tyr, and Phe. Without being bound by theory, it is speculated that the P1 preference profile exhibited by SEQ ID NO: 1 provides an effective yet mild cleavage of cell surface proteins involved in surface attachment and cell-cell adhesion. As can be seen from the Examples herein, the protease of SEQ ID NO: 1 exhibits improved cell detachment compared to Accutase®. The present inventors have subsequently succeeded in constructing a recombinant host cell capable of expressing SEQ ID NO: 1, which enables biotechnological production of the main active component of Accutase® that complies with regulatory constraints imposed on clinical development and pharmaceutical production of cell therapies.

## Polypeptides

The present invention relates to a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1. In one embodiment, the polypeptide comprises, consists essentially of, or consists of SEQ ID NO: 1.

In one embodiment, the polypeptide has an increased P1 preference for Leu, Tyr, and Phe. Preferably, Leu, Tyr, and Phe are among the five most preferred amino acid residues in the P1 position. Preferably, P1 preference is determined according to Example 2 herein.

In one embodiment, the polypeptide has polypeptide having trypsin activity. Trypsin activity (EC 3.4.21.4) may be determined according to the Trypsin Activity Assay described in the Examples herein.

In one embodiment, the polypeptide has chymotrypsin activity. Chymotrypsin activity (EC 3.4.21.1) may be determined according to the Chymotrypsin Activity Assay described in the Examples herein.

In one embodiment, the polypeptide has collagenase Type I activity. Collagenase type I activity may be determined according to the Collagenase Type I Activity Assay described in the Examples herein.

In one embodiment, the polypeptide has collagenase Type IV activity. Collagenase type IV activity may be determined according to the Collagenase Type I Activity Assay described in the Examples herein.

In another aspect, the polypeptide is derived from SEQ ID NO: 1 by substitution, deletion or addition of one or several amino acids. In some embodiments, the polypeptide is a variant of SEQ ID NO: 1 comprising a substitution, deletion, and/or insertion at one or more positions. In one aspect, the number of amino acid substitutions, deletions and/or insertions introduced into the polypeptide of SEQ ID NO: 1 is up to 15, *e.g.*, 1, 2, 3, 4, 5, 6, 7, 8, 9, 10, 11, 12, 13, 14, or 15. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding module.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant molecules are tested for protease activity and/or P1 preference to identify amino acid residues that are critical to the activity and/or the specificity of the molecule (see also Hilton et al., 1996, J. Biol. Chem. 271: 4699-4708). The active site of a polypeptide can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904;

Wlodaver et al., 1992, FEBS Lett. 309: 59-64. The identity of essential amino acids can also be inferred from an alignment with a related polypeptide, and/or be inferred from sequence homology and conserved catalytic machinery with a related polypeptide or within a polypeptide or protein family with polypeptides/proteins descending from a common ancestor, typically having similar three-dimensional structures, functions, and significant sequence similarity. Additionally, or alternatively, protein structure prediction tools can be used for protein structure modelling to identify essential amino acids and/or active sites of polypeptides. See, for example, Jumper et al., 2021, "Highly accurate protein structure prediction with AlphaFold", Nature 596: 583-589.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; US 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

In one aspect, the polypeptide is isolated.

In another aspect, the polypeptide is purified.

**Compositions Suitable for Cell Detachment**

The present invention also relates to a composition suitable for cell detachment comprising a polypeptide having protease activity a sequence identity of at least 70%, e.g., at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1, and wherein said polypeptide is present in an amount of at least 50%, e.g., at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material of the composition.

In one embodiment, the polypeptide is present in an amount of at least 90%, e.g., at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material present in the composition.

In a preferred embodiment, the polypeptide is present in an amount of at least 99%, e.g., at least 99.5%, least 99.9%, at least 99.95%, at least 99.99%, at least 99.995%, at least 99.999%, or more, by weight of the total polypeptide material present in the composition.

In a more preferred embodiment, the polypeptide is present in an amount of at least 99.9%, e.g., at least 99.95%, at least 99.99%, at least 99.995%, at least 99.999%, or more, by weight of the total polypeptide material present in the composition.

In a most preferred embodiment, the polypeptide is present in an amount of at least 99.99%, *e.g.*, at least 99.995%, at least 99.999%, or more, by weight of the total polypeptide material present in the composition.

In one embodiment, the polypeptide has an increased P1 preference for Leu, Tyr, and Phe. Preferably, Leu, Tyr, and Phe are among the five most preferred amino acid residues in the P1 position. Preferably, P1 preference is determined according to Example 2 herein.

In one embodiment, the polypeptide has trypsin activity. Trypsin activity (EC 3.4.21.4) may be determined according to the Trypsin Activity Assay described in the Examples herein.

In one embodiment, the polypeptide has chymotrypsin activity. Chymotrypsin activity (EC 3.4.21.1) may be determined according to the Chymotrypsin Activity Assay described in the Examples herein.

In one embodiment, the polypeptide has collagenase Type I activity. Collagenase type I activity may be determined according to the Collagenase Type I Activity Assay described in the Examples herein.

In one embodiment, the polypeptide has collagenase Type IV activity. Collagenase type IV activity may be determined according to the Collagenase Type I Activity Assay described in the Examples herein.

In one aspect, the composition suitable for cell detachment is a liquid composition. Preferably, the composition is an aqueous composition in order to ensure compatibility with media commonly used for cell cultures. In some embodiments, the liquid composition is freeze-dried. In another aspect, the composition is a solid composition, preferably a freeze-dried composition.

To ensure that the liquid composition has a pH value that is compatible with cell culturing conditions, the composition may comprise an aqueous buffer. The composition may comprise aqueous buffer in an amount of 1-99% by weight, e.g., 5-95%, 10-90%, 15-85%, 20-80%, or 25-75% aqueous buffer. Alternatively, the composition may comprise at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, at least 99.5%, or more, aqueous buffer by weight.

In some embodiments, the liquid composition has a pH value of about 5 to about 9, e.g., pH 5, pH 5.5, pH 6, pH 6.5, pH 7, pH 7.5, pH 8, pH 8.5, or pH 9. More preferably, the composition has a pH value of about 7 to about 8, e.g., pH 7, pH 7.1, pH 7.2, pH 7.3, pH 7.4, pH 7.5, pH 7.6, pH 7.7, pH, 7.8, pH 7.9, or pH 8. Even more preferably, the composition has a pH value of about 7 to about 7.5, e.g., pH 7.1, pH 7.2, pH 7.3, pH 7.4, or pH 7.5. Most preferably, the composition has a pH value of about 7.4.

In some embodiments, the aqueous buffer comprises 4-(2-hydroxyethyl)-1-piperazineethanesulfonic acid (HEPES), tris(hydroxymethyl)aminomethane (TRIS), phosphate, or bicarbonate. Preferably, the aqueous buffer is a HEPES buffer, a TRIS buffer, or a phosphate (e.g., PBS) buffer.

In some embodiments, the liquid composition comprises a polypeptide of the invention in an amount of from about 0.1 $\mu$g/ml to about 100 $\mu$g/ml, e.g., from about 0.5 $\mu$g/ml to about 50 $\mu$g/ml, from about 1 $\mu$g/ml to about 20 $\mu$g/ml, or from about 1 $\mu$g/ml to about 10 $\mu$g/ml.

In some embodiments, the liquid composition comprises a polypeptide of the invention in an amount of from about 0.1 $\mu$g/ml to about 20 $\mu$g/ml, e.g., about 0.1 $\mu$g/ml, about 0.2 $\mu$g/ml, about 0.3 $\mu$g/ml, about 0.4 $\mu$g/ml, about 0.5 $\mu$g/ml, about 0.6 $\mu$g/ml, about 0.7 $\mu$g/ml, about 0.8 $\mu$g/ml, about 0.9 $\mu$g/ml, about 1 $\mu$g/ml, about 2 $\mu$g/ml, about 3 $\mu$g/ml, about 4 $\mu$g/ml about 5 $\mu$g/ml, about 6 $\mu$g/ml, about 7 $\mu$g/ml, about 8 $\mu$g/ml, about 9 $\mu$g/ml, about 10 $\mu$g/ml, about 11 $\mu$g/ml, about 12 $\mu$g/ml, about 13 $\mu$g/ml, about 14 $\mu$g/ml, about 15 $\mu$g/ml, about 16 $\mu$g/ml, about 17 $\mu$g/ml, about 18 $\mu$g/ml, about 19 $\mu$g/ml, or about 20 $\mu$g/ml.

In some embodiments, the liquid composition comprises a polypeptide of the invention in an amount of from about 0.5 $\mu$g/ml to about 5 $\mu$g/ml, e.g., about 0.5 $\mu$g/ml, about 0.6 $\mu$g/ml, about 0.7 $\mu$g/ml, about 0.8 $\mu$g/ml, about 0.9 $\mu$g/ml, about 1 $\mu$g/ml, about 2 $\mu$g/ml, about 3 $\mu$g/ml, or about 4 $\mu$g/ml, or about 5 $\mu$g/ml.

In some embodiments, the liquid composition comprises a polypeptide of the invention in an amount of from about 1 $\mu$g/ml to about 10 $\mu$g/ml, e.g., about 1 $\mu$g/ml, about 2 $\mu$g/ml, about 3 $\mu$g/ml, about 4 $\mu$g/ml, about 5 $\mu$g/ml, about 6 $\mu$g/ml, about 7 $\mu$g/ml, about 8 $\mu$g/ml, about 9 $\mu$g/ml, or about 10 $\mu$g/ml.

In some embodiments, the liquid composition comprises a polypeptide of the invention in an amount of from about 1 $\mu$g/ml to about 20 $\mu$g/ml, e.g., about 1 $\mu$g/ml, about 2 $\mu$g/ml, about 3 $\mu$g/ml, about 4 $\mu$g/ml, about 5 $\mu$g/ml, about 6 $\mu$g/ml, about 7 $\mu$g/ml, about 8 $\mu$g/ml, about 9 $\mu$g/ml, about 10 $\mu$g/ml, about 11 $\mu$g/ml, about 12 $\mu$g/ml, about 13 $\mu$g/ml, about 14 $\mu$g/ml, about 15 $\mu$g/ml, about 16 $\mu$g/ml, about 17 $\mu$g/ml, about 18 $\mu$g/ml, about 19 $\mu$g/ml, or about 20 $\mu$g/ml.

In a preferred embodiment, the liquid composition comprises a polypeptide of the invention in an amount of from 1 $\mu$g/ml to 20 $\mu$g/ml, e.g., 1 $\mu$g/ml, 2 $\mu$g/ml, 3 $\mu$g/ml, 4 $\mu$g/ml, 5 $\mu$g/m, 6 $\mu$g/ml, 7 $\mu$g/ml, 8 $\mu$g/ml, 9 $\mu$g/ml, 10 $\mu$g/ml, 11 $\mu$g/ml, 12 $\mu$g/ml, 13 $\mu$g/ml, 14 $\mu$g/ml, 15 $\mu$g/ml, 16 $\mu$g/ml, 17 $\mu$g/ml, 18 $\mu$g/ml, 19 $\mu$g/ml, or 20 $\mu$g/ml, more preferably from 1 $\mu$g/ml to 10 $\mu$g/ml, most preferably from 1 $\mu$g/ml to 5 $\mu$g/ml.

In some embodiments, the liquid composition comprises ethylenediaminetetraacetic acid (EDTA). Preferably, the liquid composition comprises EDTA in an amount of from about 0.01 mM to about 100 mM, e.g., from about 0.05 mM to about 50 mM, from about 0.1 mM to about 10 mM, or from about 0.5 mM to about 5 mM. Preferably, the liquid composition comprises EDTA in an amount of about 0.1 mM, about 0.2 mM, about 0.3 mM, about 0.3 mM, about 0.4 mM, about 0.5 mM, about 0.6 mM, about 0.7 mM, about 0.8 mM, about 0.9 mM, about 0.95 mM, about 1 mM, about 1.5 mM, about 2 mM, about 3 mM, about 4 mM, about 5 mM, about 6 mM, about 7 mM, about 8 mM, about 9 mM, or about 10 mM. More preferably, liquid composition comprises EDTA in an amount of about 0.5 mM, about 0.6 mM, about 0.7 mM, about 0.8 mM, about 0.9 mM, about 0.95 mM, about 1 mM, about 1.5 mM, about 2 mM, about 3 mM, about 4 mM, or about 5 mM. Most preferably, the liquid composition comprises EDTA in an amount of about 1 mM.

In some embodiments, the liquid composition comprises substantially no magnesium ions ($Mg^{2+}$) and/or calcium ions ($Ca^{2+}$). In some embodiments, the liquid composition does not comprise magnesium ions ($Mg^{2+}$) and/or calcium ions ($Ca^{2+}$). In some embodiments, the liquid does not comprise magnesium ions ($Mg^{2+}$) or calcium ions ($Ca^{2+}$).

In some embodiments, the liquid composition comprises a phosphate buffer (e.g., PBS), EDTA, and substantially no magnesium ions ($Mg^{2+}$) and/or calcium ions ($Ca^{2+}$).

In a preferred embodiment, the liquid composition comprises a phosphate buffer (e.g., PBS) having a pH value of about 7 to about 8, preferably of about 7 to about 7.5, most preferably of about pH 7.4; wherein the liquid composition further comprises EDTA in an amount of from about 0.1 mM to about 10 mM, preferably from about 0.5 mM to about 5 mM, most preferably of about 1 mM; and wherein the liquid composition comprises substantially no magnesium ions ($Mg^{2+}$) and/or calcium ions ($Ca^{2+}$).

In a preferred embodiment, the liquid composition comprises a phosphate buffer (e.g., PBS) having a pH value of about 7 to about 7.5, most preferably of about pH 7.4; wherein the liquid composition further comprises EDTA in an amount of from about 0.5 mM to about 5 mM, most preferably of about 1 mM; and wherein the liquid composition does not comprise magnesium ions ($Mg^{2+}$) or calcium ions ($Ca^{2+}$).

In a preferred embodiment, the liquid composition comprises a phosphate buffer (e.g., PBS) having a pH value of about 7 to about 7.5, most preferably of about pH 7.4; wherein the liquid composition further comprises EDTA in an amount of from about 0.5 mM to about 5 mM, most preferably of about 1 mM; wherein the liquid composition does not comprise magnesium ions ($Mg^{2+}$) or calcium ions ($Ca^{2+}$); and wherein the composition comprises SEQ ID NO: 1 in an amount of from 0.1 μg/ml to 20 μg/ml.

In a preferred embodiment, the liquid composition comprises a phosphate buffer (e.g., PBS) having a pH value of about pH 7.4; wherein the composition further comprises EDTA in an amount of about 1 mM; and wherein the composition does not comprise magnesium ions ($Mg^{2+}$) or calcium ions ($Ca^{2+}$).

In a preferred embodiment, the liquid composition comprises a phosphate buffer (e.g., PBS) having a pH value of about pH 7.4; wherein the composition further comprises EDTA in an amount of about 1 mM; wherein the composition does not comprise magnesium ions ($Mg^{2+}$) or calcium ions ($Ca^{2+}$); and wherein the composition comprises SEQ ID NO: 1 in an amount of from 1 μg/ml to 20 μg/ml.

The liquid composition may further comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

In some embodiments, filler(s) or carrier material(s) are included to increase the volume of the liquid compositions. Suitable filler or carrier materials include, but are not limited to, various salts of sulfate, carbonate and silicate as well as talc, clay and the like. Suitable filler or carrier materials for liquid compositions include, but are not limited to, water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials.

In an aspect, the liquid composition comprises 20-80% w/w of polyol. In one embodiment, the liquid composition comprises 0.001-2% w/w preservative.

In another embodiment, the invention relates to liquid compositions comprising:

(a) 0.001-25% w/w of a polypeptide of the present invention (e.g., SEQ ID NO: 1);

(b) 20-80% w/w of polyol;

(c) optionally 0.001-2% w/w preservative; and

(d) water.

In another embodiment, the invention relates to liquid compositions comprising:

(a) 0.001-25% w/w of a polypeptide of the present invention (e.g., SEQ ID NO: 1);

(b) 0.001-2% w/w preservative;

(c) optionally 20-80% w/w of polyol; and

(d) water.

In another embodiment, the liquid composition comprises one or more formulating agents, such as a formulating agent selected from the group consisting of polyol, sodium chloride, sodium benzoate, potassium sorbate, sodium sulfate, potassium sulfate, magnesium sulfate, sodium thiosulfate, calcium carbonate, sodium citrate, dextrin, glucose, sucrose, sorbitol, lactose, starch, PVA, acetate and phosphate, preferably selected from the group consisting of sodium sulfate, dextrin, cellulose, sodium thiosulfate, kaolin and calcium carbonate. In one embodiment, the polyols is selected from the group consisting of glycerol, sorbitol, propylene glycol (MPG), ethylene glycol, diethylene glycol, triethylene glycol, 1,2-propylene glycol or 1,3-propylene glycol, dipropylene glycol, polyethylene glycol (PEG) having an average molecular weight below about 600 and polypropylene glycol (PPG) having an average molecular weight below about 600, more preferably selected from the group consisting of glycerol, sorbitol and propylene glycol (MPG) or any combination thereof.

In one embodiment, the liquid composition comprises glucose in an amount of from about 0.1 g/L to about 10 g/L, e.g., about 0.1 g/L, about 0.2 g/L, about 0.3 g/L, about 0.4 g/L, about 0.5 g/L, about 0.6 g/L, about 0.7 g/L, about 0.8 g/L, about 0.9 g/L, about 1 g/L, about 2 g/L, about 3 g/L, about 4 g/L, about 5 g/L, about 6 g/L, about 7 g/L, about 8 g/L, about 9 g/L, or about 10 g/L. In a preferred embodiment, the liquid composition comprises glucose in an amount of from about 0.5 g/L to about 5 g/L, most preferably in an amount of about 1 g/L.

In another embodiment, the liquid composition comprises 20-80% polyol (i.e., total amount of polyol), e.g., 25-75% polyol, 30-70% polyol, 35-65% polyol, or 40-60% polyol. In one embodiment, the liquid formulation comprises 20-80% polyol, e.g., 25-75% polyol, 30-70% polyol, 35-65% polyol, or 40-60% polyol, wherein the polyol is selected from the group consisting of glycerol, sorbitol, propylene glycol (MPG), ethylene glycol, diethylene glycol, triethylene glycol, 1,2-propylene glycol or 1,3-propylene glycol, dipropylene glycol, polyethylene glycol (PEG) having an average molecular weight below about 600 and polypropylene glycol (PPG) having an average molecular weight below about 600. In one embodiment, the liquid formulation comprises 20-80% polyol (i.e., total amount of polyol), e.g., 25-75% polyol, 30-70% polyol, 35-65% polyol, or 40-60% polyol, wherein the polyol is selected from the group consisting of glycerol, sorbitol and propylene glycol (MPG).

In another embodiment, the preservative is selected from the group consisting of sodium sorbate, potassium sorbate, sodium benzoate and potassium benzoate or any combination thereof. In one embodiment, the liquid compositions comprise 0.02-1.5% w/w preservative, e.g., 0.05-1% w/w preservative or 0.1-0.5% w/w preservative. In one embodiment, the liquid formulation composition 0.001-2% w/w preservative (i.e., total amount of preservative), e.g., 0.02-1.5% w/w preservative, 0.05-1% w/w preservative, or 0.1-0.5% w/w preservative, wherein the preservative is selected from the group consisting of sodium sorbate, potassium sorbate, sodium benzoate and potassium benzoate or any combination thereof.

In one aspect, the composition further comprises one or more additional enzymes, e.g., hydrolase, isomerase, ligase, lyase, oxidoreductase, and transferase. The one or more additional enzymes are preferably selected from the group consisting of acetylxylan esterase, acylglycerol lipase, amylase, alpha-amylase, beta-amylase, arabinofuranosidase, cellobiohydrolases, cellulase, DNase, feruloyl esterase, galactanase, alpha-galactosidase, beta-galactosidase, beta-glucanase, beta-glucosidase, lysophospholipase, lysozyme, alpha-mannosidase, beta-mannosidase (mannanase), phytase, phospholipase A1, phospholipase A2, phospholipase D, pullulanase, pectin esterase, triacylglycerol lipase, xylanase, beta-xylosidase or any combination thereof. In a preferred embodiment, the composition further comprises a DNAse.

**Polynucleotides**

The present invention also relates to polynucleotides encoding a polypeptide of the present invention. The polynucleotide may be a genomic DNA, a cDNA, a synthetic DNA, a synthetic RNA, a mRNA, or a combination thereof.

The polynucleotide may also be mutated by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, e.g., Ford et al., 1991, Protein Expression and Purification 2: 95-107.

In an aspect, the polynucleotide is isolated.

In another aspect, the polynucleotide is purified.

**Nucleic Acid Constructs**

The present invention also relates to nucleic acid constructs comprising a polynucleotide of the present invention, wherein the polynucleotide is operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. Techniques for modifying polynucleotides utilizing recombinant DNA methods are well known in the art.

Promoters

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the polynucleotide of the present invention in a bacterial host cell are described in Sambrook et al., 1989, Molecular Cloning: A Laboratory Manual, Cold Spring Harbor Lab., NY, Davis et al., 2012, supra, and Song et al., 2016, PLOS One 11(7): e0158447.

Examples of suitable promoters for directing transcription of the polynucleotide of the present invention in a filamentous fungal host cell are promoters obtained from *Aspergillus, Fusarium, Rhizomucor and Trichoderma* cells, such as the promoters described in Mukherjee et al., 2013, "Trichoderma: Biology and Applications", and by Schmoll and Dattenböck, 2016, "Gene Expression Systems in Fungi: Advancements and Applications", Fungal Biology.

For expression in a yeast host, examples of useful promoters are described by Smolke et al., 2018, "Synthetic Biology: Parts, Devices and Applications" (Chapter 6: Constitutive and Regulated Promoters in Yeast: How to Design and Make Use of Promoters in S. cerevisiae), and by Schmoll and Dattenböck, 2016, "Gene Expression Systems in Fungi: Advancements and Applications", Fungal Biology.

Terminators

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells may be obtained from the genes for *Bacillus clausii* alkaline protease (aprH), *Bacillus licheniformis* alpha-amylase (amyL), and *Escherichia coli* ribosomal RNA (rrnB).

Preferred terminators for filamentous fungal host cells may be obtained from *Aspergillus* or *Trichoderma* species, such as obtained from the genes for *Aspergillus niger* glucoamylase, *Trichoderma reesei* beta-glucosidase, *Trichoderma reesei* cellobiohydrolase I, and *Trichoderma reesei* endoglucanase I, such as the terminators described in Mukherjee et al., 2013, "Trichoderma: Biology and Applications", and by Schmoll and Dattenböck, 2016, "Gene Expression Systems in Fungi: Advancements and Applications", Fungal Biology.

Preferred terminators for yeast host cells may be obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992, Yeast 8: 423-488.

mRNA Stabilizers

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis* cryIIIA gene (WO 94/25612) and a *Bacillus subtilis* SP82 gene (Hue et al., 1995, J. Bacteriol. 177: 3465-3471).

Examples of mRNA stabilizer regions for fungal cells are described in Geisberg et al., 2014, Cell 156(4): 812-824, and in Morozov et al., 2006, Eukaryotic Cell 5(11): 1838-1846.

Leader Sequences

The control sequence may also be a leader, a non-translated region of an mRNA that is important for translation by the host cell. The leader is operably linked to the 5'-terminus of the polynucleotide encoding the polypeptide. Any leader that is functional in the host cell may be used.

Suitable leaders for bacterial host cells are described by Hambraeus et al., 2000, Microbiology 146(12): 3051-3059, and by Kaberdin and Bläsi, 2006, FEMS Microbiol. Rev. 30(6): 967-979.

Preferred leaders for filamentous fungal host cells may be obtained from the genes for *Aspergillus oryzae* TAKA amylase and *Aspergillus nidulans* triose phosphate isomerase.

Suitable leaders for yeast host cells may be obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

Polyadenylation Sequences

The control sequence may also be a polyadenylation sequence, a sequence operably linked to the 3'-terminus of the polynucleotide which, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus* niger alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990

Signal Peptides

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is heterologous to the coding sequence. A heterologous signal peptide coding sequence may be required where the coding sequence does not naturally contain a signal peptide coding sequence. Alternatively, a heterologous signal peptide coding sequence may simply replace the natural signal peptide coding sequence to enhance secretion of the polypeptide. Any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA. Further signal peptides are described by Freudl, 2018, Microbial Cell Factories 17: 52.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase, such as the signal peptide described by Xu et al., 2018, Biotechnology Letters 40: 949-955.

Useful signal peptides for yeast host cells are obtained from the genes for *Saccharomyces cerevisiae* alpha-factor and *Saccharomyces cerevisiae* invertase. Other useful signal peptide coding sequences are described by Romanos et al., 1992, supra.

Propeptides

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence. Additionally, or alternatively, when both signal peptide and propeptide sequences are present, the polypeptide may comprise only a part of the signal peptide sequence and/or only a part of the propeptide sequence. Alternatively, the final or isolated polypeptide may comprise a mixture of mature polypeptides and polypeptides which comprise, either partly or in full length, a propeptide sequence and/or a signal peptide sequence.

Regulatory Sequences

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac*, *tac*, and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In fungal systems, these regulatory sequences include the dihydrofolate reductase gene that is amplified in the presence of methotrexate, and the metallothionein genes that are amplified with heavy metals.

Transcription Factors

The control sequence may also be a transcription factor, a polynucleotide encoding a polynucleotide-specific DNA-binding polypeptide that controls the rate of the transcription of genetic information from DNA to mRNA by binding to a specific polynucleotide sequence. The transcription factor may function alone and/or together with one or more other polypeptides or transcription factors in a complex by promoting or blocking the recruitment of RNA polymerase. Transcription factors are characterized by comprising at least one DNA-binding domain which often attaches to a specific DNA sequence adjacent to the genetic elements which are regulated by the transcription factor. The transcription factor may regulate the expression of a protein of interest either directly, *i.e.*, by activating the transcription of the gene encoding the protein of interest by binding to its promoter, or indirectly, *i.e.*, by activating the transcription of a further transcription factor which regulates the transcription of the gene encoding the protein of interest, such as by binding to the promoter of the further transcription factor. Suitable transcription factors for fungal host cells are described in WO 2017/144177. Suitable transcription factors for prokaryotic host cells are described in Seshasayee et al., 2011, Subcellular Biochemistry 52: 7-23, as well in Balleza et al., 2009, FEMS Microbiol. Rev. 33(1): 133-151.

**Expression Vectors**

The present invention also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

The vector preferably contains at least one element that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous recombination, such as homology-directed repair (HDR), or non-homologous recombination, such as non-homologous end-joining (NHEJ).

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

More than one copy of a polynucleotide of the present invention may be inserted into a host cell to increase production of a polypeptide. For example, 2 or 3 or 4 or 5 or more copies are inserted into a host cell. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

**Recombinant Host Cells**

The present invention also relates to recombinant host cells comprising a polynucleotide of the present invention operably linked to one or more control sequences that direct the production of a polypeptide of the present invention.

Thus, in one aspect, the recombinant host cell comprises a polynucleotide encoding a polypeptide having protease activity and a sequence identity of at least 70%, e.g., at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1. In a preferred embodiment, the recombinant host cell comprises a polynucleotide encoding a polypeptide comprising, consisting essentially of, or consisting of SEQ ID NO: 1.

A construct or vector comprising a polynucleotide is introduced into a host cell so that the construct or vector is maintained as a chromosomal integrant or as a self-replicating extrachromosomal vector as described earlier. The choice of a host cell will to a large extent depend upon the gene encoding the polypeptide and its source. The polypeptide can be native or heterologous to the recombinant host cell. Also, at least one of the one or more control sequences can be heterologous to the polynucleotide encoding the polypeptide. The recombinant host cell may comprise a single copy, or at least two copies, e.g., three, four, five, or more copies of the polynucleotide of the present invention.

The host cell may be any microbial cell useful in the recombinant production of a polypeptide of the present invention, e.g., a prokaryotic cell or a fungal cell.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The prokaryotic host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. In an embodiment, the *Bacillus* cell is a *Bacillus amyloliquefaciens, Bacillus licheniformis,* or *Bacillus subtilis* cell.

In a preferred embodiment, the recombinant host cell is a *Bacillus licheniformis* cell.

In a preferred embodiment, the recombinant host cell is a *Bacillus subtilis* cell.

For purposes of this invention, *Bacillus* classes/genera/species shall be defined as described in Patel and Gupta, 2020, Int. J. Syst. Evol. Microbiol. 70: 406-438.

The bacterial host cell may also be any *Streptococcus* cell including, but not limited to, *Streptococcus equisimilis, Streptococcus pyogenes, Streptococcus uberis,* and *Streptococcus equi* subsp. *zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

Methods for introducing DNA into prokaryotic host cells are well-known in the art, and any suitable method can be used including but not limited to protoplast transformation, competent cell transformation, electroporation, conjugation, transduction, with DNA introduced as linearized or as circular polynucleotide. Persons skilled in the art will be readily able to identify a suitable method for introducing DNA into a given prokaryotic cell depending, *e.g.,* on the genus. Methods for introducing DNA into prokaryotic host cells are for example described in Heinze et al., 2018, BMC Microbiology 18:56, Burke et al., 2001, Proc. Natl. Acad. Sci. USA 98: 6289-6294, Choi et al., 2006, J. Microbiol. Methods 64: 391-397, and Donald et al., 2013, J. Bacteriol. 195(11): 2612-2620.

The host cell may be a fungal cell. "Fungi" as used herein includes the phyla Ascomycota, Basidiomycota, Chytridiomycota, and Zygomycota as well as the Oomycota and all mito-sporic fungi (as defined by Hawksworth et al., In, Ainsworth and Bisby's Dictionary of The Fungi, 8th edition, 1995, CAB International, University Press, Cambridge, UK).

Fungal cells may be transformed by a process involving protoplast-mediated transformation, Agrobacterium-mediated transformation, electroporation, biolistic method and shockwave-mediated transformation as reviewed by Li et al., 2017, Microbial Cell Factories 16: 168 and procedures described in EP 238023, Yelton et al., 1984, Proc. Natl. Acad. Sci. USA 81: 1470-1474, Christensen et al., 1988, Bio/Technology 6: 1419-1422, and Lubertozzi and Keasling, 2009, Biotechn. Advances 27: 53-75. However, any method known in the art for introducing DNA into a fungal host cell can be used, and the DNA can be introduced as linearized or as circular polynucleotide.

The fungal host cell may be a yeast cell. "Yeast" as used herein includes ascosporogenous yeast (Endomycetales), basidiosporogenous yeast, and yeast belonging to the Fungi Imperfecti (Blastomycetes). For purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell. In a preferred embodiment, the yeast host cell is a *Pichia* or *Komagataella* cell, *e.g.,* a *Pichia pastoris* cell (*Komagataella phaffii*).

In a preferred embodiment, the recombinant host cell is a *Pichia pastoris* (*Komagataella phaffii*) cell.

The fungal host cell may be a filamentous fungal cell. "Filamentous fungi" include all filamentous forms of the subdivision Eumycota and Oomycota (as defined by Hawksworth et al., 1995, supra). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell. In a preferred embodiment, the filamentous fungal host cell is an *Aspergillus, Trichoderma* or *Fusarium* cell. In a further preferred embodiment, the filamentous fungal host cell is an *Aspergillus niger, Aspergillus oryzae, Trichoderma reesei,* or *Fusarium venenatum* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In a preferred embodiment, the recombinant host cell is an *Aspergillus niger cell.*

In a preferred embodiment, the recombinant host cell is an *Aspergillus oryzae* cell.

In a preferred embodiment, the recombinant host cell is a *Trichoderma reesei* cell.

In an aspect, the recombinant host cell is isolated.

In another aspect, the recombinant host cell is purified.

**Methods of Production**

The present invention also relates to methods of producing a polypeptide of the present invention, comprising (a) cultivating a host cell, which in its wild-type form produces a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1 under conditions conducive for production of the polypeptide; and optionally, (b) recovering the polypeptide. In a preferred embodiment, the polypeptide comprises, consists essentially of, or consists of SEQ ID NO: 1.

The present invention also relates to methods of producing a polypeptide of the present invention, comprising (a) cultivating a recombinant host cell of the present invention under conditions conducive for production of a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1; and optionally, (b) recovering the polypeptide. In a preferred embodiment, the polypeptide comprises, consists essentially of, or consists of SEQ ID NO: 1.

In one aspect, the recombinant host cell is a prokaryotic host cell, preferably a bacterial cell. In one embodiment, the recombinant host cell is a *Bacillus* cell, preferably a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* cells. In a preferred embodiment, the recombinant host cell is a *Bacillus amyloliquefaciens, Bacillus licheniformis,* or *Bacillus subtilis* cell.

In a preferred embodiment, the recombinant host cell is a *Bacillus licheniformis* cell.

In a preferred embodiment, the recombinant host cell is a *Bacillus subtilis* cell.

In one aspect, the recombinant host cell is a filamentous fungal host cell. In one embodiment, the recombinant host cell is selected from the group consisting of *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium*

*venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* and *Trichoderma viride* cell. In a preferred embodiment, the recombinant host cell is an *Aspergillus niger, Aspergillus oryzae,* or *Trichoderma reesei* cell. In a preferred embodiment, the recombinant host cell is an *Aspergillus niger* cell.

In a preferred embodiment, the recombinant host cell is an *Aspergillus niger cell.*

In a preferred embodiment, the recombinant host cell is an *Aspergillus oryzae* cell.

In a preferred embodiment, the recombinant host cell is a *Trichoderma reesei* cell.

In one aspect, the recombinant host cell is a yeast host cell. In one embodiment, the recombinant host cell is selected from the group consisting of *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell. In a preferred embodiment, the yeast host cell is a *Pichia* or *Komagataella* cell, *e.g.*, a *Pichia pastoris* cell (*Komagataella phaffii*).

In a preferred embodiment, the recombinant host cell is a *Pichia pastoris* (*Komagataella phaffii*) cell.

The host cell or the recombinant host cell is cultivated in a nutrient medium suitable for production of the polypeptide using methods known in the art. For example, the cell may be cultivated by shake flask cultivation, or small-scale or large-scale fermentation (including continuous, batch, fed-batch, or solid-state, and/or microcarrier-based fermentations) in laboratory or industrial fermentors in a suitable medium and under conditions allowing the polypeptide to be expressed and/or isolated. Suitable media are available from commercial suppliers or may be prepared according to published compositions (*e.g.*, in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptide, including, but not limited to, the use of specific antibodies, formation of an enzyme product, disappearance of an enzyme substrate, or an assay determining the relative or specific activity of the polypeptide.

The polypeptide may be recovered from the medium using methods known in the art, including, but not limited to, collection, centrifugation, filtration, extraction, spray-drying, freeze-drying, evaporation, or precipitation. In one aspect, a whole fermentation broth comprising the polypeptide is recovered. In another aspect, a cell-free fermentation broth comprising the polypeptide is recovered.

The polypeptide may be purified by a variety of procedures known in the art to obtain substantially pure polypeptides and/or polypeptide fragments (see, *e.g.*, Wingfield, 2015, Current Protocols in Protein Science; 80(1): 6.1.1-6.1.35; Labrou, 2014, Protein Downstream Processing, 1129: 3-10).

In an alternative aspect, the polypeptide is not recovered.

**Protease Granules**

The present invention also relates to enzyme granules/particles comprising a polypeptide of the present invention, *i.e.*, a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1. In one embodiment, the polypeptide comprises, consists essentially of, or consists of SEQ ID NO: 1.

In an embodiment, the granule comprises a core, and optionally one or more coatings (outer layers) surrounding the core.

The core may have a diameter, measured as equivalent spherical diameter (volume based average particle size), of 20-2000 μm, particularly 50-1500 μm, 100-1500 μm or 250-1200 μm. The core diameter, measured as equivalent spherical diameter, can be determined using laser diffraction, such as using a Malvern Mastersizer and/or the method described under ISO 13320 (2020).

In an embodiment, the core comprises a polypeptide of the present invention.

The core may include additional materials such as fillers, fiber materials (cellulose or synthetic fibers), stabilizing agents, solubilizing agents, suspension agents, viscosity regulating agents, light spheres, plasticizers, salts, lubricants and fragrances.

The core may include a binder, such as synthetic polymer, wax, fat, or carbohydrate.

The core may include a salt of a multivalent cation, a reducing agent, an antioxidant, a peroxide decomposing catalyst and/or an acidic buffer component, typically as a homogenous blend.

The core may include an inert particle with the polypeptide absorbed into it, or applied onto the surface, *e.g.*, by fluid bed coating.

The core may have a diameter of 20-2000 $\mu$m, particularly 50-1500 $\mu$m, 100-1500 $\mu$m or 250-1200 $\mu$m.

The core may be surrounded by at least one coating, *e.g.*, to improve the storage stability, to reduce dust formation during handling, or for coloring the granule. The optional coating(s) may include a salt coating, or other suitable coating materials, such as polyethylene glycol (PEG), methyl hydroxy-propyl cellulose (MHPC) and polyvinyl alcohol (PVA).

The coating may be applied in an amount of at least 0.1% by weight of the core, e.g., at least 0.5%, at least 1%, at least 5%, at least 10%, or at least 15%. The amount may be at most 100%, 70%, 50%, 40% or 30%.

The coating is preferably at least 0.1 $\mu$m thick, particularly at least 0.5 $\mu$m, at least 1 $\mu$m or at least 5 $\mu$m. In some embodiments, the thickness of the coating is below 100 $\mu$m, such as below 60 $\mu$m, or below 40 $\mu$m.

The coating should encapsulate the core unit by forming a substantially continuous layer. A substantially continuous layer is to be understood as a coating having few or no holes, so that the core unit has few or no uncoated areas. The layer or coating should, in particular, be homogeneous in thickness.

The coating can further contain other materials as known in the art, *e.g.*, fillers, antisticking agents, pigments, dyes, plasticizers and/or binders, such as titanium dioxide, kaolin, calcium carbonate or talc.

A salt coating may comprise at least 60% by weight of a salt, *e.g.*, at least 65%, at least 70%, at least 75%, at least 80%, at least 85%, at least 90%, at least 95% or at least 99% by weight.

To provide acceptable protection, the salt coating is preferably at least 0.1 $\mu$m thick, *e.g.*, at least 0.5 $\mu$m, at least 1 $\mu$m, at least 2 $\mu$m, at least 4 $\mu$m, at least 5 $\mu$m, or at least 8 $\mu$m. In a particular embodiment, the thickness of the salt coating is below 100 $\mu$m, such as below 60 $\mu$m, or below 40 $\mu$m.

The salt may be added from a salt solution where the salt is completely dissolved or from a salt suspension wherein the fine particles are less than 50 $\mu$m, such as less than 10 $\mu$m or less than 5 $\mu$m.

The salt coating may comprise a single salt or a mixture of two or more salts. The salt may be water soluble, in particular, having a solubility at least 0.1 g in 100 g of water at 20°C, preferably at least 0.5 g per 100 g water, *e.g.*, at least 1 g per 100 g water, *e.g.*, at least 5 g per 100 g water.

The salt may be an inorganic salt, *e.g.*, salts of sulfate, sulfite, phosphate, phosphonate, nitrate, chloride or carbonate or salts of simple organic acids (less than 10 carbon atoms, *e.g.*, 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminum. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular, alkali- or earth alkali metal salts of sulfate, sulfite, phosphate, phosphonate, nitrate, chloride or carbonate or salts of simple organic acids such as citrate, malonate or acetate may be used.

The salt in the coating may have a constant humidity at 20°C above 60%, particularly above 70%, above 80% or above 85%, or it may be another hydrate form of such a salt (*e.g.*, anhydrate). The salt coating may be as described in WO 00/01793 or WO 2006/034710.

Specific examples of suitable salts are NaCl (CH20°C=76%), $Na_2CO_3$ (CH20°C=92%), $NaNO_3$ (CH20°C=73%), $Na_2HPO_4$ (CH20°C=95%), $Na_3PO_4$ (CH25°C=92%), $NH_4Cl$ (CH20°C = 79.5%), $(NH_4)_2HPO_4$ (CH20°C = 93,0%), $NH_4H_2PO_4$ (CH20°C = 93.1%), $(NH_4)_2SO_4$ (CH20°C=81.1%), KCl (CH20°C=85%), $K_2HPO_4$ (CH20°C=92%), $KH_2PO_4$ (CH20°C=96.5%), $KNO_3$ (CH20°C=93.5%), $Na_2SO_4$ (CH20°C=93%), $K_2SO_4$ (CH20°C=98%), $KHSO_4$ (CH20°C=86%), $MgSO_4$ (CH20°C=90%), $ZnSO_4$ (CH20°C=90%) and sodium citrate (CH25°C=86%). Other examples include $NaH_2PO_4$, $(NH_4)H_2PO_4$, $CuSO_4$, $Mg(NO_3)_2$ and magnesium acetate.

The salt may be in anhydrous form, or it may be a hydrated salt, *i.e.*, a crystalline salt hydrate with bound water(s) of crystallization, such as described in WO 99/32595. Specific examples include anhydrous sodium sulfate ($Na_2SO_4$), anhydrous magnesium sulfate ($MgSO_4$), magnesium sulfate heptahydrate ($MgSO_4 \cdot 7H_2O$), zinc sulfate heptahydrate ($ZnSO_4 \cdot 7H_2O$), sodium phosphate dibasic heptahydrate ($Na_2HPO_4 \cdot 7H_2O$), magnesium nitrate hexahydrate ($Mg(NO_3)_2(6H_2O)$), sodium citrate dihydrate and magnesium acetate tetrahydrate.

Preferably the salt is applied as a solution of the salt, *e.g.*, using a fluid bed.

The coating materials can be waxy coating materials and film-forming coating materials. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591.

The granule may optionally have one or more additional coatings. Examples of suitable coating materials are polyethylene glycol (PEG), methyl hydroxy-propyl cellulose (MHPC) and polyvinyl alcohol (PVA). Examples of enzyme granules with multiple coatings are described in WO 93/07263 and WO 97/23606.

The core can be prepared by granulating a blend of the ingredients, *e.g.*, by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation.

Methods for preparing the core can be found in the Handbook of Powder Technology; Particle size enlargement by C. E. Capes; Vol. 1; 1980; Elsevier. Preparation methods include known feed and granule formulation technologies, e.g.,

(a) Spray dried products, wherein a liquid polypeptide-containing solution is atomized in a spray drying tower to form small droplets which during their way down the drying tower dry to form a polypeptide-containing particulate material. Very small particles can be produced this way (Michael S. Showell (editor); Powdered detergents; Surfactant Science Series; 1998; Vol. 71; pages 140-142; Marcel Dekker).

(b) Layered products, wherein the polypeptide is coated as a layer around a pre-formed inert core particle, wherein a polypeptide-containing solution is atomized, typically in a fluid bed apparatus wherein the pre-formed core particles are fluidized, and the polypeptide-containing solution adheres to the core particles and dries up to leave a layer of dry polypeptide on the surface of the core particle. Particles of a desired size can be obtained this way if a useful core particle of the desired size can be found. This type of product is described in, e.g., WO 97/23606.

(c) Absorbed core particles, wherein rather than coating the polypeptide as a layer around the core, the polypeptide is absorbed onto and/or into the surface of the core. Such a process is described in WO 97/39116.

(d) Extrusion or pelletized products, wherein a polypeptide-containing paste is pressed to pellets or under pressure is extruded through a small opening and cut into particles which are subsequently dried. Such particles usually have a considerable size because of the material in which the extrusion opening is made (usually a plate with bore holes) sets a limit on the allowable pressure drop over the extrusion opening. Also, very high extrusion pressures when using a small opening increase heat generation in the polypeptide paste, which is harmful to the polypeptide (Michael S. Showell (editor); Powdered detergents; Surfactant Science Series; 1998; Vol. 71; pages 140-142; Marcel Dekker).

(e) Prilled products, wherein a polypeptide-containing powder is suspended in molten wax and the suspension is sprayed, e.g., through a rotating disk atomizer, into a cooling chamber where the droplets quickly solidify (Michael S. Showell (editor); Powdered detergents; Surfactant Science Series; 1998; Vol. 71; pages 140-142; Marcel Dekker). The product obtained is one wherein the polypeptide is uniformly distributed throughout an inert material instead of being concentrated on its surface. US 4,016,040 and US 4,713,245 describe this technique.

(f) Mixer granulation products, wherein a polypeptide-containing liquid is added to a dry powder composition of conventional granulating components. The liquid and the powder in a suitable proportion are mixed and as the moisture of the liquid is absorbed in the dry powder, the components of the dry powder will start to adhere and agglomerate and particles will build up, forming granulates comprising the polypeptide. Such a process is described in US 4,106,991, EP 170360, EP 304332, EP 304331, WO 90/09440 and WO 90/09428. In a particular aspect of this process, various high-shear mixers can be used as granulators. Granulates consisting of polypeptide, fillers and binders etc. are mixed with cellulose fibers to reinforce the particles to produce a so-called T-granulate. Reinforced particles are more robust and release less enzymatic dust.

(g) Size reduction, wherein the cores are produced by milling or crushing of larger particles, pellets, tablets, briquettes etc. containing the polypeptide. The wanted core particle fraction is obtained by sieving the milled or crushed product. Over and undersized particles can be recycled. Size reduction is described in Martin Rhodes (editor); Principles of Powder Technology; 1990; Chapter 10; John Wiley & Sons.

(h) Fluid bed granulation. Fluid bed granulation involves suspending particulates in an air stream and spraying a liquid onto the fluidized particles via nozzles. Particles hit by spray droplets get wetted and become tacky. The tacky particles collide with other particles and adhere to them to form a granule.

(i) The cores may be subjected to drying, such as in a fluid bed drier. Other known methods for drying granules in the feed or enzyme industry can be used by the skilled person. The drying preferably takes place at a product temperature of from 25 to 90°C. For some polypeptides, it is important the cores comprising the polypeptide contain a low amount of water before coating with the salt. If water sensitive polypeptides are coated with a salt before excessive water is removed, the excessive water will be trapped within the core and may affect the activity of the polypeptide negatively. After drying, the cores preferably contain 0.1-10% w/w water.

Non-dusting granulates may be produced, e.g., as disclosed in US 4,106,991 and US 4,661,452 and may optionally be coated by methods known in the art.

The granulate may further comprise one or more additional enzymes, e.g., hydrolase, isomerase, ligase, lyase, oxidoreductase, and transferase. The one or more additional enzymes are preferably selected from the group consisting of acetylxylan esterase, acylglycerol lipase, amylase, alpha-amylase, beta-amylase, arabinofuranosidase, cellobiohydrolases, cellulase, feruloyl esterase, galactanase, alpha-galactosidase, beta-galactosidase, beta-glucanase, beta-glucosidase, lysophospholipase, lysozyme, alpha-mannosidase, beta-mannosidase (mannanase), phytase, phospholipase A1, phospholipase A2, phospholipase D, protease, pullulanase, pectin esterase, triacylglycerol lipase, xylanase, beta-xylosidase or any combination thereof. Each enzyme will then be present in more granules securing a more uniform distribution of the enzymes, and also reduces the physical segregation of different enzymes due to different particle sizes. Methods for producing multi-enzyme co-granulates is disclosed in the ip.com disclosure IP-COM000200739D.

Another example of formulation of polypeptides by the use of co-granulates is disclosed in WO 2013/188331.

The present invention also relates to protected polypeptides prepared according to the method disclosed in EP 238216.

**Fermentation Broth Formulations or Cell Compositions**

The present invention also relates to a fermentation broth formulation or a cell composition comprising a polypeptide of the present invention, *i.e.*, a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1. In one embodiment, the polypeptide comprises, consists essentially of, or consists of SEQ ID NO: 1.

The fermentation broth formulation or the cell composition further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.*, filamentous fungal cells) are removed, *e.g.*, by centrifugation. In some embodiments, the fermentation broth contains spent cell culture medium, extracellular enzymes, and viable and/or nonviable microbial cells.

In some embodiments, the fermentation broth formulation or the cell composition comprises a first organic acid component comprising at least one 1-5 carbon organic acid and/or a salt thereof and a second organic acid component comprising at least one 6 or more carbon organic acid and/or a salt thereof. In some embodiments, the first organic acid component is acetic acid, formic acid, propionic acid, a salt thereof, or a mixture of two or more of the foregoing and the second organic acid component is benzoic acid, cyclohexanecarboxylic acid, 4-methyl-valeric acid, phenylacetic acid, a salt thereof, or a mixture of two or more of the foregoing.

In one aspect, the composition contains an organic acid(s), and optionally further contains killed cells and/or cell debris. In some embodiments, the killed cells and/or cell debris are removed from a cell-killed whole broth to provide a composition that is free of these components.

The fermentation broth formulation or cell composition may further comprise a preservative and/or anti-microbial (*e.g.*, bacteriostatic) agent, including, but not limited to, sorbitol, sodium chloride, potassium sorbate, and others known in the art.

The cell-killed whole broth or cell composition may contain the unfractionated contents of the fermentation materials derived at the end of the fermentation. Typically, the cell-killed whole broth or cell composition contains the spent culture medium and cell debris present after the microbial cells (*e.g.*, filamentous fungal cells) are grown to saturation, incubated under carbon-limiting conditions to allow protein synthesis. In some embodiments, the cell-killed whole broth or cell composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673

**Methods and Uses**

The present invention also relates to methods for cell detachment, comprising contacting a cell with a polypeptide of the invention or a composition comprising a polypeptide of the invention, wherein the cell is attached to a surface and/or to another cell. In one embodiment, the cell to be detached is attached to a surface. In one embodiment, the cell to be detached is part of a cell monolayer. In one embodiment, the cell to be detached is part of a cell cluster.

In one embodiment, the polypeptide of the invention has protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1. In one embodiment, the polypeptide comprises, consists essentially of, or consists of SEQ ID NO: 1.

In one embodiment, the polypeptide has an increased P1 preference for Leu, Tyr, and Phe. Preferably, Leu, Tyr, and Phe are among the five most preferred amino acid residues in the P1 position. Preferably, P1 preference is determined according to Example 2 herein.

Methods of the present invention may be used to detach any type of cell including, but not limited to, A375 metastatic melanoma cells, beta cells, BHK cells, bone marrow stem cells, cardiomyocytes, CHO cells, COS cells, D54 glioma cells, dopaminergic progenitor cells, fibroblasts, HEK293 cells, HeLa cells, hepatocytes, hepatocyte progenitor cells, human stem cells, HT1080 fibrosarcoma cells, immortalized mouse testicular germ cells, keratinocytes, L929 cells, M24 metastatic melanoma cells, macrophages, MG63 cells, NIH/3T3 cells, NT2 cells, primary chick embryo neuronal cells, Sf9 insect cells, U251 glioma cells, vascular endothelial cells, vascular smooth muscle cells, and Vero cells.

In a preferred embodiment, the cell is a mammalian cell, most preferably a human cell.

Methods of the present invention may be used to detach any type of stem cell or stem cell derivative. Thus, the stem cell may be a totipotent stem cell (*e.g.*, a fertilized egg cell), a pluripotent stem cell (*e.g.*, an embryonic stem cell), a multipotent stem cell (*e.g.*, a mesenchymal stem cell), an oligopotent stem cell (*e.g.*, a hematopoietic stem cell), or a unipotent stem cell (*e.g.*, a muscle stem cell). In one embodiment, the stem cell is a human stem cell. In one embodiment, the stem cell is a human pluripotent stem cell, a human multipotent stem cell, a human oligopotent stem cell, or a human unipotent stem cell. In a preferred embodiment, the stem cell is a human pluripotent stem cell. In another preferred embodiment, the stem cell is a human induced pluripotent stem cell.

In some embodiments, the cell to be detached is attached to a surface, e.g., a plastic surface or a glass surface. In some embodiments, the cell to be detached is attached to another cell. In some embodiments, the cell to be attached is part of a cell cluster.

In some embodiments, the cell to be detached is attached to a surface coated with biomaterials, extra cellular matrix (ECM), and/or other scaffolds fabricated from natural polymers (*e.g.*, collagen, hyaluronic acid, fibrin, alginate, gelatine, etc.) or synthetic polymers (*e.g.*, poly(glycolic acid) (PGA), poly(lactic acid) (PLA), poly(lactic-co-glycolic acid) (PLGA), and polycaprolactone (PCL), etc.).

In one embodiment, the cell to be detached is a pluripotent stem cell, preferably a human pluripotent stem cell. Preferably, the pluripotent stem cell is detached from a surface, preferably a plastic surface or a glass surface, or from a cell cluster.

In one embodiment, the cell to be detached is an induced pluripotent stem cell, preferably a human induced pluripotent stem cell. Preferably, the induced pluripotent stem cell is detached from a surface, preferably a plastic surface or a glass surface, or from a cell cluster.

In one embodiment, the cell to be detached is a cardiomyocyte, preferably a human cardiomyocyte. Preferably, the cardiomyocyte is detached from a surface, preferably a plastic surface or a glass surface, or from a cell cluster.

In one embodiment, the cell to be detached is a stem cell-derived cardiomyocyte, preferably a human stem cell-derived cardiomyocyte. Preferably, the stem cell-derived cardiomyocyte is detached from a surface, preferably a plastic surface or a glass surface, or from a cell cluster.

The present invention also relates to use of a polypeptide of the invention in a cell detachment process. In one embodiment, the polypeptide of the invention has protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1. In one embodiment, the polypeptide comprises, consists essentially of, or consists of SEQ ID NO: 1.

In one embodiment, the polypeptide has an increased P1 preference for Leu, Tyr, and Phe. Preferably, Leu, Tyr, and Phe are among the five most preferred amino acid residues in the P1 position. Preferably, P1 preference is determined according to Example 2 herein.

Polypeptides of the invention may be used in any type of cell detachment process including, but not limited to, detachment of A375 metastatic melanoma cells, beta cells, BHK cells, bone marrow stem cells, cardiomyocytes, CHO cells, COS cells, D54 glioma cells, dopaminergic progenitor cells, fibroblasts, HEK293 cells, HeLa cells, hepatocytes, hepatocyte progenitor cells, human stem cells, HT1080 fibrosarcoma cells, immortalized mouse testicular germ cells, keratinocytes, L929 cells, M24 metastatic melanoma cells, macrophages, MG63 cells, NIH/3T3 cells, NT2 cells, primary chick embryo neuronal cells, Sf9 insect cells, U251 glioma cells, vascular endothelial cells, vascular smooth muscle cells, and Vero cells.

In a preferred embodiment, the cell to be detached is a mammalian cell, most preferably a human cell.

Polypeptides of the invention may be used in any type of stem cell detachment process. Thus, the stem cell to be detached may be a totipotent stem cell (*e.g.*, a fertilized egg cell), a pluripotent stem cell (*e.g.*, an embryonic stem cell), a multipotent stem cell (*e.g.*, a mesenchymal stem cell), an oligopotent stem cell (*e.g.*, a hematopoietic stem cell), or a unipotent stem cell (*e.g.*, a muscle stem cell). In one embodiment, the stem cell is a human stem cell. In one embodiment, the stem cell is a human pluripotent stem cell, a human multipotent stem cell, a human oligopotent stem cell, or a human unipotent stem cell. In a preferred embodiment, the stem cell is a human pluripotent stem cell. In another preferred embodiment, the stem cell is a human induced pluripotent stem cell.

In one embodiment, the cell to be detached is a stem cell derivative, preferably a pluripotent stem cell derivative, most preferably a human pluripotent stem cell derivative.

In some embodiments, the cell to be detached is attached to a surface, e.g., a plastic surface or a glass surface. In some embodiments, the cell to be detached is attached to another cell. In some embodiments, the cell to be attached is part of a cell cluster.

In some embodiments, the cell to be detached is attached to a surface coated with biomaterials, extra cellular matrix (ECM), and/or other scaffolds fabricated from natural polymers (*e.g.*, collagen, hyaluronic acid, fibrin, alginate, gelatine, etc.) or synthetic polymers (*e.g.*, poly(glycolic acid) (PGA), poly(lactic acid) (PLA), poly(lactic-co-glycolic acid) (PLGA), and polycaprolactone (PCL), etc.).

In one embodiment, the cell to be detached is a pluripotent stem cell, preferably a human pluripotent stem cell. Preferably, the pluripotent stem cell is detached from a surface, preferably a plastic surface or a glass surface, or from a cell cluster.

In one embodiment, the cell to be detached is an induced pluripotent stem cell, preferably a human induced pluripotent stem cell. Preferably, the induced pluripotent stem cell is detached from a surface, preferably a plastic surface or a glass surface, or from a cell cluster.

In one embodiment, the cell to be detached is a cardiomyocyte, preferably a human cardiomyocyte. Preferably, the cardiomyocyte is detached from a surface, preferably a plastic surface or a glass surface, or from a cell cluster.

In one embodiment, the cell to be detached is a stem cell-derived cardiomyocyte, preferably a human stem cell-derived cardiomyocyte. Preferably, the stem cell-derived cardiomyocyte is detached from a surface, preferably a plastic surface or a glass surface, or from a cell cluster.

**Preferred embodiments**

1) A composition suitable for cell detachment comprising a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1, and wherein said polypeptide is present in an amount of at least 50%, *e.g.*, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material of the composition.

2) The composition according to embodiment 1, wherein the polypeptide has an increased P1 preference for Leu, Tyr, and Phe; preferably wherein Leu, Tyr, and Phe are among the five most preferred amino acid residues in the P1 position.

3) The composition according to any of the preceding embodiments, wherein the polypeptide comprises, consists essentially of, or consists of SEQ ID NO: 1.

4) The composition according to any of the preceding embodiments, wherein the polypeptide is a variant of fragment of SEQ ID NO: 1.

5) The composition according to any of the preceding embodiments, wherein the polypeptide is present in an amount of at least 90%, *e.g.*, at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material of the composition.

6) The composition according to any of the preceding embodiments, which is a liquid composition; preferably an aqueous composition.

7) The composition according to embodiment 6, wherein the liquid composition comprises an aqueous buffer; preferably wherein the aqueous buffer comprises 4-(2-hydroxyethyl)-1-piperazineethanesulfonic acid (HEPES), tris(hydroxymethyl)aminomethane (TRIS), phosphate, or bicarbonate; most preferably wherein the aqueous buffer comprises phosphate or bicarbonate.

8) The composition according to any of embodiments 6-7, which has a pH value of about 5 to about 9; more preferably of about 7 to about 8; most preferably of about 7 to about 7.5.

9) The composition according to any of embodiments 6-8, which comprises EDTA; preferably the composition comprises EDTA in an amount of from about 0.1 mM to about 10 mM.

10) The composition according to any of embodiments 6-9, which comprises substantially no magnesium ions ($Mg^{2+}$) and/or calcium ions ($Ca^{2+}$); preferably wherein the composition does not comprise magnesium ions ($Mg^{2+}$) or calcium ions ($Ca^{2+}$).

11) The composition according to any of embodiments 6-10, wherein the polypeptide is present in an amount of from about 0.1 $\mu$g/ml to about 20 $\mu$g/ml; preferably in an amount of from about 1 $\mu$g/ml to about 20 $\mu$g/ml.

12) An isolated polynucleotide encoding a polypeptide having at least 70% sequence identity to SEQ ID NO: 1; preferably wherein the polynucleotide encodes a polypeptide comprising, consisting of, or consisting essentially of SEQ ID NO: 1.

13) A nucleic acid construct or an expression vector comprising a polynucleotide encoding a polypeptide having at least 70% sequence identity to SEQ ID NO: 1 operably linked to a heterologous promoter; preferably wherein the polynucleotide encodes a polypeptide comprising, consisting of, or consisting essentially of SEQ ID NO: 1.

14) A recombinant host cell comprising in its genome the nucleic acid construct or expression vector of embodiment 13.

15) The recombinant host cell of embodiment 14, which is a *B. subtilis* cell, *B. licheniformis* cell, *A. niger* cell, *A. oryzae* cell, *T. reesei* cell, or *P. pastoris* (*K. phaffii*) cell.

16) A method of producing a polypeptide having a sequence identity of at least 70% to SEQ ID NO: 1 and having protease activity, comprising (a) cultivating a recombinant host cell according to any of embodiments 14-15 under conditions conducive for expression of the polypeptide; and optionally, (b) recovering the polypeptide.

17) The method according to embodiment 16, wherein the polypeptide comprises, consists essentially of, or consists of SEQ ID NO: 1.

18) A granule or particle comprising a polypeptide having a sequence identity of at least 70% to SEQ ID NO: 1 and having protease activity; preferably wherein the polypeptide comprises, consists essentially of, or consists of SEQ ID NO 1.

19) A fermentation broth formulation comprising a polypeptide having a sequence identity of at least 70% to SEQ ID NO: 1 and having protease activity; preferably wherein the polypeptide comprises, consists essentially of, or consists of SEQ ID NO 1.

20) A method for cell detachment, comprising contacting a cell with a polypeptide having protease activity and a sequence identity of at least 70%, *e.g.*, at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1 or a composition comprising said polypeptide, wherein the cell is attached to a surface and/or to another cell.

21) The method according to embodiment 20, wherein the polypeptide has an increased P1 preference for Leu, Tyr, and Phe; preferably wherein Leu, Tyr, and Phe are among the five most preferred amino acid residues in the P1 position.

22) The method according to any of embodiments 20-21, wherein the polypeptide comprises, consists essentially of, or consists of SEQ ID NO: 1.

23) The method according to any of embodiments 20-22, wherein the polypeptide is a variant of fragment of SEQ ID NO: 1.

24) The method according to any of embodiments 20-23, wherein the polypeptide has a purity of least 90%, *e.g.*, at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material present in the composition.

25) The method according to any of embodiments 20-24, wherein the composition is a liquid composition; preferably an aqueous composition.

26) The method according to embodiment 25, wherein the liquid composition comprises an aqueous buffer; preferably wherein the aqueous buffer comprises 4-(2-hydroxyethyl)-1-piperazineethanesulfonic acid (HEPES), tris(hydroxymethyl)aminomethane (TRIS), phosphate, or bicarbonate; most preferably wherein the aqueous buffer comprises phosphate or bicarbonate.

27) The method according to any of embodiments 25-26, wherein the liquid composition has a pH value of about 5 to about 9; more preferably of about 7 to about 8; most preferably of about 7 to about 7.5.

28) The method according to any of embodiments 25-27, wherein the liquid composition comprises EDTA; preferably the composition comprises EDTA in an amount of from about 0.1 mM to about 10 mM.

29) The method according to any of embodiments 25-28, wherein the liquid composition comprises substantially no magnesium ions ($Mg^{2+}$) and/or calcium ions ($Ca^{2+}$); preferably wherein the composition does not comprise magnesium ions ($Mg^{2+}$) or calcium ions ($Ca^{2+}$).

30) The method according to any of embodiments 25-29, wherein the liquid composition comprises the polypeptide in an amount of from about 0.1 µg/ml to about 20 µg/ml; preferably wherein the liquid composition comprises the polypeptide in an amount of from about 1 µg/ml to about 20 µg/ml.

31) The method according to any of embodiments 20-20, wherein the cell is a human cell.

32) The method according to any of embodiments 20-31, wherein the cell is a stem cell or a stem cell derivative; preferably wherein the cell is a pluripotent stem cell, an induced pluripotent stem cell, or a (stem cell-derived) cardiomyocyte.

33) The method according to any of embodiments 20-32, wherein the surface is a plastic surface or a glass surface.

34) Use of a polypeptide having protease activity and a sequence identity of at least 70%, e.g., at least 75%, at least 80%, at least 85%, at least 90%, at least 91%, at least 92%, at least 93%, at least 94%, at least 95%, at least 96%, at least 97%, at least 98%, at least 99%, or 100%, to SEQ ID NO: 1 or a composition comprising said polypeptide in a cell detachment process.

35) The use according to embodiment 34, wherein the polypeptide has an increased P1 preference for Leu, Tyr, and Phe; preferably wherein Leu, Tyr, and Phe are among the five most preferred amino acid residues in the P1 position.

36) The use according to any of embodiments 34-35, wherein the polypeptide comprises, consists essentially of, or consists of SEQ ID NO: 1.

37) The use according to any of embodiments 34-36, wherein the polypeptide is a variant of fragment of SEQ ID NO: 1.

38) The use according to any of embodiments 34-37, wherein the polypeptide has a purity of at least 90%, e.g., at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material present in the composition.

39) The use according to any of embodiments 34-38, wherein the composition is a liquid composition; preferably an aqueous composition.

40) The use according to embodiment 39, wherein the liquid composition comprises an aqueous buffer; preferably wherein the aqueous buffer comprises 4-(2-hydroxyethyl)-1-piperazineethanesulfonic acid (HEPES), tris(hydroxymethyl)aminomethane (TRIS), phosphate, or bicarbonate; most preferably wherein the aqueous buffer comprises phosphate or bicarbonate.

41) The use according to any of embodiments 39-40, wherein the liquid composition has a pH value of about 5 to about 9; more preferably of about 7 to about 8; most preferably of about 7 to about 7.5.

42) The use according to any of embodiments 39-41, wherein the liquid composition comprises EDTA; preferably the composition comprises EDTA in an amount of from about 0.1 mM to about 10 mM.

43) The use according to any of embodiments 39-42, wherein the liquid composition comprises substantially no magnesium ions ($Mg^{2+}$) and/or calcium ions ($Ca^{2+}$); preferably wherein the composition does not comprise magnesium ions ($Mg^{2+}$) or calcium ions ($Ca^{2+}$).

44) The use according to any of embodiments 39-43, wherein the liquid composition comprises the polypeptide in an amount of from about 0.1 µg/ml to about 20 µg/ml; preferably wherein the liquid composition comprises the polypeptide in an amount of from about 1 µg/ml to about 20 µg/ml.

45) The use according to any of embodiments 34-44, wherein the cell is a human cell.

46) The use according to any of embodiments 34-45, wherein the cell is a stem cell or a stem cell derivative; preferably wherein the cell is a pluripotent stem cell, an induced pluripotent stem cell, or a (stem cell-derived) cardiomyocyte.

47) The use according to any embodiments 34-46, wherein the cell is detached from a surface or from a cell cluster.

48) The use according to embodiment 47, wherein the surface is a plastic surface or a glass surface.

## EXAMPLES

### Materials and methods

#### Desalting of Accutase

Lyophilized Accutase® XL (Sigma Aldrich, full amount) was dissolved in 25 mL MilliQ water, and 10 mL was loaded to a HiPrep 26/10 desalting chromatographic column (Sigma Aldrich) equilibrated with 50 mM Tricine, 10 mM

CaCl$_2$, pH 7.5 at a flowrate of 10 mL/min at 10 °C using an ÄKTAexplorer 100. Elution continued in the same buffer. The first peak fraction was collected and the protein concentration was determined from the absorbance at 280 nm (A$_{280}$) and stored at -20 °C. Desalted Accutase was used for proteolytic activity assays (trypsin, chymotrypsin, collagenase type I, and collagenase type IV).

Trypsin Activity Assay

The assay was conducted in a 96-well format using a total well volume of 200 μL. The enzyme concentrations ranged from 0.5-200 μg/mL. The substrate (Nα-Benzoyl-L-arginine ethyl ester, BAEE, Merck) was diluted in 67 mM phosphate buffer, pH 7.5. The final BAEE substrate solution was 0.28 mg/mL. Samples were diluted in 67 mM phosphate buffer, pH 7.5 (sodium phosphate dibasic heptahydrate 13.544 g, sodium phosphate monobasic monohydrate 2.274 g, add milliQ water to 1 L). Samples were tested in a concentration range from 5 to 100 μg/mL. 15 μL diluted sample was added to 185 μL BAEE substrate solution. The reaction was monitored over 10 minutes by measuring the absorbance at 253 nm (A253) at 27 °C.

Chymotrypsin Activity Assay

The assay was conducted in a 96-well format using a total well volume of 200 μL. The enzyme concentrations ranged from 20-500 μg/mL. The substrate (Nα-Benzoyl-L-tyrosine ethyl ester, BTEE, Sigma) was dissolved in 1 mL 96% ethanol and diluted in 4 mL 80 mM Tris (2-Amino-2-(hydroxymethyl)-1,3-propanediol) with 17 mM beta-cyclodextrin, pH 7.5. The final BTEE substrate solution was 1.72 mg/mL. Samples were diluted in 80 mM Tris pH 7.5 (assay buffer). Samples were tested in a concentration range from 50 to 500 μg/mL. 20 μL diluted sample was added to 120 μL assay buffer and 60 μL BTEE substrate solution. The reaction was monitored over 10 minutes by measuring the absorbance at 256 nm (A256) at 27 °C.

Collagenase Type I Activity Assay

30 μL 1 mg/mL collagen FITC (Merck) suspended in 1 mM acetic acid, 20 μL 0.25-7.5 μg/mL enzyme in DPBS (Dulbecco's phosphate buffered saline without Ca$^{2+}$ and Mg$^{2+}$, Merck) and 100 μL 0.5 M Tricine (Sigma) pH 7.4 was mixed. The reaction was conducted at 37 °C and was stopped by adding 150 μL ice cold DPBS after 30, 60 or 90 min followed by centrifugation (13000$g$; 5 min). The reaction was quantified by measuring excitation at 485 nm and emission (detection) at 535 nm on 100 μL supernatant in duplicates.

Collagenase Type IV Activity Assay

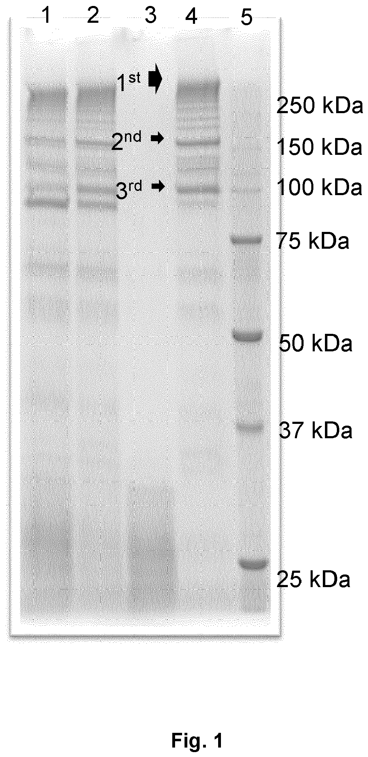

The enzymes were diluted to 1, 0.25 and 0.1 mg/mL in 100 mM Tricine pH 7.4. and mixed with 40 μL 5 mg/mL collagen Type IV (human placenta, Sigma-Aldrich) and pH adjusted using 10 μL, 0.5 M Tricine pH 7.4. Reaction was stopped after 15 min by adding 105 μl 10% Trichloroacetic acid precipitation and incubated for 10 min at 5°C and subsequently centrifuged (13000$g$; 3 min). The supernatant was removed, and the precipitate was solubilized in 50 μL sample buffer (200 μL 4× Laemmli Sample buffer (Bio-Rad item no. 161-0747), 40 μL reducing agent (Bio-Rad), 30 μL 2M Tris and 130 μL milliQ water). The samples were subjected to SDS-PAGE analysis using gel, Tris/Glycine/SDS buffer, and Precision Plus Protein ladder from Bio-Rad. The activity of the enzymes was scored as having no (0) activity, lower (-), equal (+) to or higher (++) activity than Accutase (desalted sample). For an example, see Figure 1 where degradation of the first band at >200 kDa, the second band at 150 kDa and the third band at 100 kDa were observed.

Protease Activity Assay

The proteolytic activity of polypeptide can be determined by a method employing the Suc-AAPF-pNA substrate. Suc-AAPF-pNA is an abbreviation for N-Succinyl-Alanine-Alanine-Proline-Phenylalanine-p-Nitroanilide, and it is a blocked peptide which can be cleaved by endo-proteases. Following proteolytic cleavage, a free pNA molecule having a yellow color is liberated and can be measured by visible spectrophotometry at wavelength 405 nm. The Suc-AAPF-PNA substrate is manufactured, *e.g.*, by Bachem (cat. no. L1400, dissolved in DMSO).

A sample containing the polypeptide to be analyzed is diluted in residual activity buffer (100 mM Tris, pH 8.6). The assay is performed by transferring 30 μl of diluted enzyme samples to 96 well microtiter plate and adding 70 μl substrate working solution (0.72 mg/ml in 100 mM Tris, pH 8.6). The solution is mixed at room temperature and absorption is measured every 20 sec. over 5 minutes at 405 nm. The sample should be diluted to a level where the slope is linear. The slope (absorbance per minute) of the time-dependent absorption curve is directly proportional to the proteolytic activity of the polypeptide under the given set of conditions.

**Evaluation of stem cell detachment and cluster dissociation**

Evaluation of human pluripotent stem cells detachment and cluster dissociation

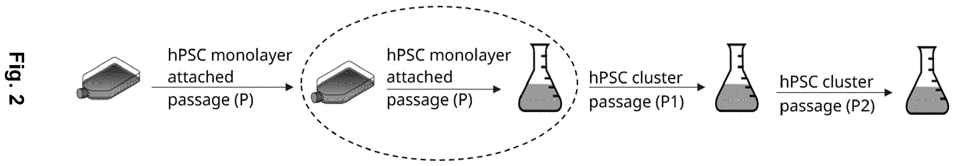

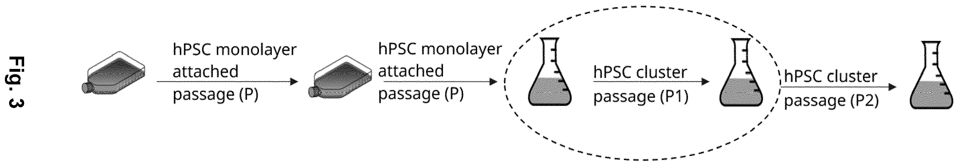

Human pluripotent stem cells (hPSCs) were grown as attached monolayers in the primary stages and as clusters in later stages of production. After detachment of hPSC monolayers, the formation of uniform clusters for suspension growth and expansion was followed. See Figures 2 and 3 for a schematic overview of the process as well as an indication of when evaluations were performed.

*Evaluation of hPSC surface detachment and cluster formation:*

Spent media was removed from the T-flask and wash buffer (PBS without $Ca^{2+}$ and $Mg^{2+}$; 0.04-0.3 mL/cm$^2$) was added. The cell monolayer was washed, and the buffer was removed. The enzyme solution was prepared by dissolving SEQ ID NO: 1 in Dulbecco's PBS buffer without $Ca^{2+}$ and $Mg^{2+}$ to a concentration of 8.3 μg/mL followed by preheating to room temperature and addition of 0.5 mM ethylenediaminetetraacetic acid (EDTA).

The enzyme solution was added to the cell culture vessels (0.04-0.3 mL/cm$^2$), and the vessels were incubated at 37 °C for 3-20 min. Cultivation media was added to the vessels and the cell suspension was mixed by pipetting to obtain a single cell suspension. After surface detachment, the cells were centrifuged, re-suspended in appropriate volume, and counted using a NucleoCounter NC202 (ChemoMetec, Denmark) and the total cell count, cell viability, and fraction of aggregates (Aggregate %) were recorded according to [1]). To estimate cluster formation efficiency, fold change on day 1 after passage was determined. Cells in clusters were determined by using the NucleoCounter NC200 (ChemoMetec, Denmark) according to [2]. To estimate cell growth, fold change per day was determined on day 3 after seeding according to [2]. The cluster size and size distribution (cluster diameter coefficient of variation) was determined using the Biorep Islet Cell Counter according to [3] and [4] on day 3 after passage. The evaluated parameters were scored according to Table 1 below, and an average score was calculated.

| Table 1: Scoring of hPSC surface detachment and cluster formation | | | | |
|---|---|---|---|---|
| Score | 0 | 1 | 2 | 3 |
| Cell viability (%) normalised to Accutase | <0.85 | 0.85-0.95 | 0.95-1.05 | >1.05 |
| Fraction of aggregates (%) | >25 | 10.1-25 | 2.1-10 | 0-2 |
| Fold change on day 1 after passage normalised to Accutase (number of re-aggregated cells on day 1 after seeding divided by number of seeded cells) | <0.70 | 0.70-0.90 | 0.91-1.10 | >1.10 |
| Fold change per day normalised to Accutase (number of cells divided by number of seeded cells divided by number of days) | <0.70 | 0.70-0.90 | 0.91-1.10 | >1.10 |
| Cluster diameter coefficient of variation (%) on day 3 after passage, normalised to Accutase | >1.45 | 1.15-1.45 | 0.85-1.15 | <0.85 |

*Evaluation of hPSC cluster dissociation and cluster re-formation:*

The hPSC clusters were centrifuged and washed (PBS without $Ca^{2+}$ and $Mg^{2+}$; 0.1-1 mL per mL of original working volume, typically 0.25 mL/mL). The enzyme solutions were prepared by dissolving SEQ ID NO: 1 in Dulbecco's PBS buffer without $Ca^{2+}$ and $Mg^{2+}$ to a concentration of 4 μg/mL followed by preheating to room temperature and addition of 0.5 mM EDTA.

Enzyme solution was then added to the cells (0.025-0.5 mL per mL of working volume; typically, 0.2 mL/mL), and the cells were incubated at 37 °C for 3-15 min. Cultivation media was added to the vessels and the cell suspension was mixed by pipetting to obtain a single cell suspension. After cluster dissociation, the cells were centrifuged, re-suspended in appropriate volume, and counted using a NucleoCounter NC202 (ChemoMetec, Denmark) and the total cell count, cell viability, and fraction of aggregates (Aggregate %) were recorded according to [1]). To estimate cluster formation efficiency, fold change on day 1 after passage was determined. Cells in clusters were determined by using the NucleoCounter NC200 (ChemoMetec, Denmark) according to [2]. To estimate cell growth, fold change per day was determined on day 3 after seeding according to [2]. The cluster size and size distribution (cluster diameter coefficient of variation) was determined using the Biorep Islet Cell Counter according to [3] and [4] on day 3 after passage. The evaluated parameters were scored according to Table 2 below, and an average score was calculated.

The pluripotent phenotype was confirmed after a second passage in cluster stage by staining with antibodies specific for surface markers Oct4 (BD) and Nanog (Nordic BioSite ApS) using fluorescence-activated single cell sorting (FACS) analysis.

| Table 2: Scoring of hPSC cluster dissociation and re-formation | | | | |
|---|---|---|---|---|
| Score | 0 | 1 | 2 | 3 |
| Cell viability (%) normalised to Accutase | <0.85 | 0.85-0.95 | 0.95-1.05 | >1.05 |
| Fraction of aggregates (%) | >25 | 15-25 | 2.1-10 | 0-2 |
| Fold change day 1 after passage normalised to Accutase (number of re-aggregated cells day 1 after seeding divided by number of seeded cells | <0.7 | 0.7-0.9 | 0.91-1.1 | >1.1 |
| Fold change per day normalised to Accutase (number of cells divided by number of seeded cells divided by days) | <0.7 | 0.7-0.9 | 0.91-1.1 | >1.1 |
| Cluster diameter coefficient of variation (%) on day 3 after passage, normalised to Accutase | >1.45 | 1.15-1.45 | 0.85-1.15 | <0.85 |

Evaluation of cardiomyocyte cluster dissociation

*CM08 dissociation:*

Stem cell-derived cardiomyocyte (CM) clusters were cultured in shake-flask format in RPMI medium supplemented with 1 % ascorbic acid and 2% B-27 supplement (Gibco) on a shaker incubator (70 rpm; 37 °C). At the CM08 stage (eight days after differentiation), 3 mL clusters were harvested by centrifugation (300g; 1 min) and the supernatant medium removed. The cells were subsequently washed with 3 mL PBS without $Ca^{2+}$ and $Mg^{2+}$, centrifugated (300g; 1 min) and the supernatant was removed. The enzyme solution was prepared by dissolving SEQ ID NO:1 in Dulbecco's PBS buffer without $Ca^{2+}$ and $Mg^{2+}$; to a concentration of 8 $\mu$g/mL. followed by preheating to room temperature and addition of 0.5 mM EDTA. 1 mL enzyme solution was added to the CM clusters, and the clusters were incubated (70 rpm; 37 °C) until the medium became turbid with single cells. The clusters were ensured fully disintegrated, and 2 mL culture medium (preheated to 37 °C) was added to stop the enzymatic reaction.

*CM08 analysis:*

After adding culture medium, images were taken and cell count was performed to determine total live and dead cell numbers, cell viability and remaining clusters (not fully dissociated into single-cell suspension) using the ICC4 Automatic Cell Counter (BioRep).

*Cluster re-formation culture:*

Collected cells were resuspended in culture medium for re-formation of clusters at a density of $10^6$ viable cells/mL in an incubator at 70 rpm and 37 °C. Medium was fully changed every 2-3 days and the re-formation of clusters was followed for seven days followed by harvesting of the clusters at stage CM15.

*CM15 harvest and analysis:*

Cluster number, cluster size distribution and morphology were checked using the ICC4 Automatic Cell Counter (BioRep). The clusters were then harvested and analyzed for cluster re-formation yield ([total viable cell count/total seeded viable cell count] * 100) and average cluster size estimate (pIEQ divided by total cluster number) [1, 2].

*Evaluation and scoring:*

Parameters important to dissociation and the following cluster formations were evaluated and scored (see Table 3). An average score was calculated for the enzyme solution.

| Table 3: Scoring of CM cluster dissociation | | | | |
|---|---|---|---|---|
| Parameter | 0 | 1 | 2 | 3 |
| CM08 Cell viability (%) | <90 | ≤90 and <95 | ≤95 and <105 | ≤105 |
| CM08 Remaining clusters (%) | >6 | ≤6 and >3 | ≤3 and >-3 | ≤-3 |
| CM15 Cluster re-formation yield (%) | <70 | ≤70 and <90 | ≤90 and <110 | ≤110 |
| CM15 Average cluster size | >185 | ≤185 and >115 | ≤115 and >85 | ≤85 |

**Example 1a: Purification of SEQ ID NO: 1 from Accutase**

Accutase™ XL (lyophilized, available from, e.g., Merck) was dissolved in milliQ water at room temperature, pH adjusted to 8.0, filtered and applied (flow rate 3.5 mL/min) to a Butyl Sepharose FF (Cytiva) column of volume 6.4 mL packed in a XK16/20 column (Cytiva) and equilibrated with 50 mM sodium phosphate pH 7.0 (39 mL 0.5 M $NaH_2PO_4 \cdot 2H_2O$, and 61 mL 0.5 M $Na_2HPO_4$, add 1 L MilliQ water), 1.5 M ammonium sulphate, pH 7.0. Following a wash step with the equilibration buffer, the protein was eluted by 50 mM sodium phosphate, pH 7.0, in a gradient over 40 column volumes at 5 mL/minute. Liquid chromatography-mass spectrometry (LC-MS) was used to confirm the molecular weight of SEQ ID NO: 1.

**Example 1b: Recombinant expression of SEQ ID NO: 1**

Cloning and expression of SEQ ID NO: 1 was done using the strategy described in US 2019/0225988 using three overlapping fragments for integration in the niiA/niiD (nitrite reductase/nitrate reductase) locus of the *Aspergillus oryzae* host strain ColS1300. The middle fragment, corresponding to the gene encoding SEQ ID NO: 1 was ordered as a synthetic gene codon optimized *for Aspergillus oryzae* (provided as SEQ ID NO: 2) and containing overlapping sequences to the two flanking fragments. SEQ ID NO: 1 was expressed with a beta-glucosidase secretion signal from *Aspergillus aculeatus* (amino acid sequence: MKLSWLVAAALTAASVVSA; provided as SEQ ID NO: 3) replacing the genes native secretion signal.

The three amplified overlapping fragments were transformed into COLS1300. Transformants were plated on plates with minimal media supplemented with $NaNO_3$, and the plates were incubated three days at 30 °C. Only spores where the integration cassette had successfully recombined into the chromosome of the host cell and reconstituted the niiA and niaD sites in the process could germinate and survive. A recombinant *Aspergillus oryzae* clone containing the sequence confirmed integrated expression construct was cultivated in liquid culture in a deep well plate with 24 wells (Corning) containing 2 ml YPG media (WO 05/066338) for five days at 30 °C without shaking.

The enzyme-containing supernatant was harvested and the expression of the SEQ ID NO: 1 was confirmed using MS analysis.

**Example 2: Determination of protease specificity**

Protease specificity may be defined according to P1 preference. The P1 position is defined as the amino acid residue situated N-terminally to the cleavage site of a polypeptide (Bio-chemical and Biophysical Research Communications, volume 27, issue 2, 20 April 1967, pages 157-162).

Preference is defined as the observed occurrence being relatively higher than occurrence expected with random cleavage when counting the number of cleavage sites from a protease digestion performed on a complex protein substrate including a high sequence diversity.

Specifically, a purified protease sample is incubated with Yeast Protein Extract (Promega V7341) at 37 °C in 100 mM HEPES, 1 mM $CaCl_2$, pH 7 for 16 hours on a 10 kDa cutoff spin filter. Before incubation the substrate is denatured by trichloroacetic acid (TCA) precipitation, reduced through addition of dithiothreitol (DTT), and alkylated via addition of iodoacetamide (IAA).

Three reactions in a protease:substrate ratios of 1:1250, 1:6250, 1:30000 are performed. The resulting protease digests are collected after centrifugation by collecting the flowthrough. An extra wash should be included to increase the peptide recovery. The protease digests are acidified with TFA and analyzed directly by LC-MS/MS *e.g.*, Evosep One (Evosep) / timsTOF Pro (Bruker Daltonik).

For peptide identification the data are searched against the UniProt yeast reference proteome using the Mascot search engine (Matrix science) with the following search parameters:

Enzyme: none

Peptide mass tolerance: ± 25 ppm

Fragment mass tolerance: ± 0.05 Da

Max missed cleavages: 0

Protease cleavage sites are deduced from N- and C-terminals of identified peptides. The degree of proteolysis should be low enough to reflect the initially preferred cleavage sites. From knowledge of the amino acid sequence of the proteins from which the peptides originate, amino acids present in subsites (*e.g.*, P1) upon proteolytic cleavage are identified.

Preference for an amino acid in a subsite (*e.g.*, P1) is calculated by comparing sum of intensities of identified peptides with this amino acid in the subsite (*e.g.*, P1) relative to the expected prevalence with random cleavage of the proteins.

Based on this method, the P1 preference of SEQ ID NO: 1 was evaluated. SEQ ID NO: 1 has an increased P1 preference for the amino acid residues Leu, Tyr, and Phe. When the P1 preference of SEQ ID NO: 1 is determined for all twenty canonical amino acids and subsequently ranked, Leu, Tyr, and Phe are among the five most preferred amino acid residues in the P1 position.

## Example 3: Proteolytic activity of SEQ ID NO: 1 and Accutase

The total protein concentration Accutase was determined to be 20 µg/mL. The trypsin, chymotrypsin, collagen type I, and collagen type IV activity of Accutase were evaluated and normalized to 100%, and the enzymatic activities of SEQ ID NO: 1 are reported relative to Accutase (see Table 4).

**Table 4:** Enzymatic activity of SEQ ID NO: 1 and Accutase. The trypsin, chymotrypsin and collagen type I activity data are relative to Accutase. Scoring of collagen type IV activity was based on SDS-PAGE evaluation where "-" means less than, "+" means equal to, and "++" means higher than Accutase.

| Sample | Conc. (µg/mL) | Trypsin (%) | Chymotrypsin (%) | Collagenase type I (%) | Collagenase type IV (score) |
|---|---|---|---|---|---|
| Accutase (desalt.) | 20 | 100 | 100 | 100 | + |
| SEQ ID NO: 1 | 4 | 5 | 100 | 48 | + |

## Example 4: hPSC monolayer detachment and cluster formation of SEQ ID NO: 1

SEQ ID NO: 1 and Accutase were evaluated and scored for detachment of hPSC monolayers and cluster formation (Table 5).

**Table 5:** Evaluation and scoring of hPSC monolayer detachment and cluster formation parameters following treatment with SEQ ID NO: 1 and Accutase.

| Parameter | SEQ ID NO: 1 | Accutase |
|---|---|---|
| Cell viability (%) normalised to Accutase | 2 | 2 |
| Fraction of aggregates (%) | 3 | 2 |
| Fold change on day 1 after passage normalised to Accutase (number of cells in clusters after seeding divided by number of seeded cells) | 3 | 2 |
| Fold change per day normalised to Accutase (number of cells divided by number of seeded cells divided by days) | 2 | 2 |
| Cluster diameter coefficient of variation (%) normalised to Accutase | 1* | 2 |
| Average score | 2.2 | 2 |
| *Clusters became more uniform when evaluated after cluster dissociation followed by cluster re-formation (see Table 6) | | |

The overall performance of SEQ ID NO: 1 was improved compared to Accutase. In particular, the fraction of aggregates was reduced, whereas cluster formation efficiency was improved.

**Example 5: hPSC cluster dissociation and re-formation with SEQ ID NO: 1**

SEQ ID NO: 1 and Accutase were evaluated and scored for hPSC cluster dissociation and re-formation (Table 6).

| Table 6: Evaluation and scoring of hPSC clusters dissociation and re-formation following treatment with SEQ ID NO: 1 or Accutase. | | |
| --- | --- | --- |
| Parameter | SEQ ID NO: 1 | Accutase |
| Cell viability (%) normalised to Accutase | 2 | 2 |
| Fraction of aggregates (%) | 2 | 2 |
| Fold change day 1 normalised to Accutase (number of cells in clusters on day 1 after seeding divided by number of seeded cells) | 3 | 2 |
| Fold change per day normalised to Accutase (number of cells divided by number of seeded cells divided by number of days) | 3 | 2 |
| Cluster diameter coefficient of variation (%) normalised to Accutase | 3 | 2 |
| Average score | 2.6 | 2 |

The overall performance of SEQ ID NO: 1 was improved compared to Accutase. In particular, cluster formation efficiency, cell growth, and cluster uniformity were improved.

**Example 6: Cardiomyocyte cluster dissociation and re-formation with SEQ ID NO: 1**

SEQ ID NO: 1 and Accutase were evaluated and scored for CM cluster dissociation and re-formation (Table 7).

| Table 7: Evaluation and scoring of CM clusters dissociation and re-formation following treatment with SEQ ID NO: 1 or Accutase. | | |
| --- | --- | --- |
| Parameters | SEQ ID NO: 1 | Accutase |
| CM08 Cell viability (%) | 2 | 2 |
| CM08 Remaining clusters (%) | 1 | 2 |
| CM 15 Cluster re-formation yield (%) | 3 | 2 |
| CM15 Average cluster size | 3 | 2 |
| Average score | 2.25 | 2 |

The overall performance of SEQ ID NO: 1 was improved compared to Accutase. In particular, cluster re-formation yield and average cluster size were improved.

**References**

1) ChemoMetec, Application Note No. 2026. Rev. 1.4. Count & Viability - Via2-Cassette™
2) ChemoMetec, Application Note No. 2028. Rev. 1.3. Aggregated Cells - Via2-Cassette™
3) BioRep User manual for AUTOMATIC ISLET CELL COUNTER 4, Ref. ICC-04:
4) Fully Automated Islet Cell Counter (ICC) for the Assessment of Islet Mass, Purity, and Size Distribution by Digital Image Analysis. Peter Buchwald, Andres Bernal, Felipe Echeverri, Alejandro Tamayo-Garcia, Elina Linetsky and Camillo Ricordi. Cell Transplantation. Vol. 25, pp. 1747-1761, 2016.

**Claims**

1.  A composition suitable for cell detachment comprising a polypeptide having protease activity and a sequence identity of at least 70% to SEQ ID NO: 1, wherein said polypeptide is present in an amount of at least 50% by weight of the total polypeptide material of the composition.

2.  The composition according to claim 1, wherein the polypeptide is present in an amount of at least 90%, e.g., at least 91%, at least 92%, at least 93%, at least 94%, at least 95%, at least 96%, at least 97%, at least 98%, at least 99%, at least 99.5%, at least 99.9%, at least 99.95%, at least 99.99%, at least 99.995%, at least 99.999%, or more, by weight of the total polypeptide material of the composition.

3.  The composition according to any of the preceding claims, wherein the polypeptide has an increased P1 preference for Leu, Tyr, and Phe; preferably wherein Leu, Tyr, and Phe are among the five most preferred amino acid residues in the P1 position.

4.  The composition according to any of the preceding claims, wherein the polypeptide comprises, consists essentially of, or consists of SEQ ID NO: 1.

5.  The composition according to any of the preceding claims, which is a liquid composition; preferably an aqueous composition.

6.  The composition according to claim 5, which comprises an aqueous buffer; preferably a phosphate buffer.

7.  The composition according to any of claims 5-6, which has a pH value of about 5 to about 9; more preferably of about 7 to about 8; most preferably of about 7 to about 7.5.

8.  The composition according to any of claims 5-7, which comprises EDTA; preferably the composition comprises EDTA in an amount of from about 0.1 mM to about 10 mM.

9.  The composition according to any of claims 5-8, which comprises substantially no magnesium ions ($Mg^{2+}$) and/or calcium ions ($Ca^{2+}$)

10. An isolated polynucleotide encoding a polypeptide having at least 70% sequence identity to SEQ ID NO: 1; preferably wherein the polynucleotide encodes a polypeptide comprising, consisting of, or consisting essentially of SEQ ID NO: 1.

11. A nucleic acid construct or an expression vector comprising a polynucleotide encoding a polypeptide having at least 70% sequence identity to SEQ ID NO: 1 operably linked to a heterologous promoter; preferably wherein the polynucleotide encodes a polypeptide comprising, consisting of, or consisting essentially of SEQ ID NO: 1.

12. A recombinant host cell comprising in its genome the nucleic acid construct or expression vector of claim 11; preferably wherein the recombinant host cell is a B. *subtilis* cell, B. *licheniformis* cell, *A. niger* cell, *A. oryzae* cell, *T. reesei* cell, or P. *pastoris* (*K. phaffii*) cell.

13. A method of producing a polypeptide having a sequence identity of at least 70% to SEQ ID NO: 1, comprising (a) cultivating a recombinant host cell according to claim 12 under conditions conducive for expression of the polypeptide; and optionally, (b) recovering the polypeptide.

14. A fermentation broth formulation comprising a polypeptide having a sequence identity of at least 70% to SEQ ID NO: 1; preferably wherein the polypeptide comprises, consists essentially of, or consists of SEQ ID NO 1.

15. A method for cell detachment, comprising contacting a cell with a composition according to any of claims 1-5, wherein the cell is attached to a surface and/or to another cell.

16. Use of a composition according to any of claims 1-5 in a cell detachment process.

**Fig. 1**

Fig. 2

Fig. 3

33

EP 4 389 865 A1

Europäisches Patentamt
European Patent Office
Office européen des brevets

**EUROPEAN SEARCH REPORT**

Application Number

EP 22 21 5666

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHI X Z ET AL: "Expression of four trypsin-like serine proteases from the Chinese shrimp, Fenneropenaeus chinensis, as regulated by pathogenic infection", COMPARATIVE BIOCHEMISTRY AND PHYSIOLOGY PART B: BIOCHEMISTRY AND MOLECULAR BIOLOGY, ELSEVIER, AMSTERDAM, NL, vol. 153, no. 1, 1 May 2009 (2009-05-01), pages 54-60, XP026062811, ISSN: 1096-4959, DOI: 10.1016/J.CBPB.2009.01.011 [retrieved on 2009-01-29] | 1-3,5-7, 10-14 | INV. C11D3/386 C12N5/07 C12N9/50 |
| Y | * Figure 1, discussion * | 8,9,15, 16 | |
| X | WO 2008/143679 A2 (VERENIUM CORP [US]; UNIV CALIFORNIA [US] ET AL.) 27 November 2008 (2008-11-27) | 1,3,5-7, 10-14 | |
| Y | * Claim 52; SEQ ID NO 69246 * | 8,9,15, 16 | |
| X | WO 2008/153429 A2 (UNIV JAGIELLONSKI [PL]; BIOCENTRUM SP Z O O [PL] ET AL.) 18 December 2008 (2008-12-18) | 1-3,5-7, 10-14 | TECHNICAL FIELDS SEARCHED (IPC) C11D C12N |
| Y | * Figure 2, claims * | 8,9,15, 16 | |
| Y | WO 2014/106141 A1 (JANSSEN BIOTECH INC [US]) 3 July 2014 (2014-07-03) * [0143], [0146], [0209], claims 1, 2, 3, 9 * | 8,9,15, 16 | |

The present search report has been drawn up for all claims

| Place of search | Date of completion of the search | Examiner |
|---|---|---|
| Munich | 24 May 2023 | Gómez Ortiz, Mariola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

34

## ANNEX TO THE EUROPEAN SEARCH REPORT
## ON EUROPEAN PATENT APPLICATION NO.

EP 22 21 5666

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2023

| Patent document cited in search report | | Publication date | Patent family member(s) | | Publication date |
|---|---|---|---|---|---|
| WO 2008143679 | A2 | 27-11-2008 | US | 2011296543 A1 | 01-12-2011 |
|  |  |  | WO | 2008143679 A2 | 27-11-2008 |
| WO 2008153429 | A2 | 18-12-2008 | NONE | | |
| WO 2014106141 | A1 | 03-07-2014 | AU | 2013370228 A1 | 09-07-2015 |
|  |  |  | BR | 112015015714 A2 | 11-07-2017 |
|  |  |  | CA | 2896750 A1 | 03-07-2014 |
|  |  |  | CN | 105705634 A | 22-06-2016 |
|  |  |  | DK | 2938722 T3 | 14-02-2022 |
|  |  |  | EP | 2938722 A1 | 04-11-2015 |
|  |  |  | EP | 4039798 A1 | 10-08-2022 |
|  |  |  | ES | 2906998 T3 | 21-04-2022 |
|  |  |  | HK | 1217727 A1 | 20-01-2017 |
|  |  |  | JP | 6529440 B2 | 12-06-2019 |
|  |  |  | JP | 2016504037 A | 12-02-2016 |
|  |  |  | KR | 20150103203 A | 09-09-2015 |
|  |  |  | PH | 12015501477 A1 | 21-09-2015 |
|  |  |  | RU | 2015131839 A | 07-02-2017 |
|  |  |  | RU | 2018108090 A | 25-02-2019 |
|  |  |  | SG | 10201709384S A | 30-01-2018 |
|  |  |  | SG | 11201505119U A | 30-07-2015 |
|  |  |  | US | 2014242693 A1 | 28-08-2014 |
|  |  |  | WO | 2014106141 A1 | 03-07-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9517413 A
- WO 9522625 A
- US 5223409 A
- WO 9206204 A
- WO 9425612 A
- WO 9533836 A
- WO 2017144177 A
- EP 238023 A
- WO 0001793 A
- WO 2006034710 A
- WO 9932595 A
- GB 1483591 A
- WO 9307263 A
- WO 9723606 A
- WO 9739116 A

- US 4016040 A
- US 4713245 A
- US 4106991 A
- EP 170360 A
- EP 304332 A
- EP 304331 A
- WO 9009440 A
- WO 9009428 A
- US 4661452 A
- WO 2013188331 A
- EP 238216 A
- WO 9015861 A
- WO 2010096673 A
- WO 05066338 A

### Non-patent literature cited in the description

- **NEEDLEMAN ; WUNSCH.** *J. Mol. Biol.,* 1970, vol. 48, 443-453
- **RICE et al.** *Trends Genet.,* 2000, vol. 16, 276-277
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **JUMPER et al.** Highly accurate protein structure prediction with AlphaFold. *Nature,* 2021, vol. 596, 583-589
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **FORD et al.** *Protein Expression and Purification,* 1991, vol. 2, 95-107

- **SAMBROOK et al.** Molecular Cloning: A Laboratory Manual. Cold Spring Harbor Lab, 1989
- **SONG et al.** *PLOS One,* 2016, vol. 11 (7), e0158447
- **MUKHERJEE et al.** *Trichoderma: Biology and Applications,* 2013
- **SCHMOLL ; DATTENBÖCK.** Gene Expression Systems in Fungi: Advancements and Applications. *Fungal Biology,* 2016
- Synthetic Biology: Parts, Devices and Applications. **SMOLKE et al.** Constitutive and Regulated Promoters in Yeast: How to Design and Make Use of Promoters in S. cerevisiae. 2018
- **ROMANOS et al.** *Yeast,* 1992, vol. 8, 423-488
- **HUE et al.** *J. Bacteriol.,* 1995, vol. 177, 3465-3471
- **GEISBERG et al.** *Cell,* 2014, vol. 156 (4), 812-824
- **MOROZOV et al.** *Eukaryotic Cell,* 2006, vol. 5 (11), 1838-1846
- **HAMBRAEUS et al.** *Microbiology,* 2000, vol. 146 (12), 3051-3059
- **KABERDIN ; BLÄSI.** *FEMS Microbiol. Rev.,* 2006, vol. 30 (6), 967-979
- **GUO ; SHERMAN.** *Mol. Cellular Biol.,* 1995, vol. 15, 5983-5990
- **FREUDL.** *Microbial Cell Factories,* 2018, vol. 17, 52
- **XU et al.** *Biotechnology Letters,* 2018, vol. 40, 949-955

- **SESHASAYEE et al.** *Subcellular Biochemistry,* 2011, vol. 52, 7-23
- **BALLEZA et al.** *FEMS Microbiol. Rev.,* 2009, vol. 33 (1), 133-151
- **PATEL ; GUPTA.** *Int. J. Syst. Evol. Microbiol.,* 2020, vol. 70, 406-438
- **HEINZE et al.** *BMC Microbiology,* 2018, vol. 18, 56
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **DONALD et al.** *J. Bacteriol.,* 2013, vol. 195 (11), 2612-2620
- **HAWKSWORTH et al.** Ainsworth and Bisby's Dictionary of The Fungi. CAB International, University Press, 1995
- **LI et al.** *Microbial Cell Factories,* 2017, vol. 16, 168
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **LUBERTOZZI ; KEASLING.** *Biotechn. Advances,* 2009, vol. 27, 53-75
- Soc. App. Bacteriol. Symposium Series. 1980
- **WINGFIELD.** *Current Protocols in Protein Science,* 2015, vol. 80 (1), 6.1.1-6.1.35
- **LABROU.** *Protein Downstream Processing,* 2014, vol. 1129, 3-10
- Particle size enlargement. **C. E. CAPES.** Handbook of Powder Technology. Elsevier, 1980, vol. 1
- Powdered detergents. Surfactant Science Series. Marcel Dekker, 1998, vol. 71, 140-142
- Principles of Powder Technology. John Wiley & Sons, 1990
- *Bio-chemical and Biophysical Research Communications,* 20 April 1967, vol. 27 (2), 157-162
- **PETER BUCHWALD ; ANDRES BERNAL ; FELIPE ECHEVERRI ; ALEJANDRO TAMAYO-GARCIA ; ELINA LINETSKY ; CAMILLO RICORDI.** Fully Automated Islet Cell Counter (ICC) for the Assessment of Islet Mass, Purity, and Size Distribution by Digital Image Analysis. *Cell Transplantation.,* 2016, vol. 25, 1747-1761